# EUROPEAN PATENT SPECIFICATION

(11) **EP 3 394 277 B1**
(45) Date of publication and mention of the grant of the patent: **12.11.2025**
(21) Application number: 16823134.8
(22) Date of filing: 20.12.2016
(51) Int. Cl.: C12P 19/02, C12P 19/14, C12N 15/79

(54) **IMPROVED GRANULAR STARCH CONVERSION ENZYMES AND METHODS**
VERBESSERTE ENZYME UND VERFAHREN ZUR UMWANDLUNG VON GRANULARER STÄRKE
ENZYMES ET PROCÉDÉS DE CONVERSION D'AMIDON GRANULÉ AMÉLIORÉS

(30) Priority: 21.12.2015 WO PCT/CN2015/098121
(43) Date of publication of application: 31.10.2018
(73) Proprietor: Danisco US Inc., Palo Alto, California 94304 (US); Koops, Bart C., Palo Alto, California 94304 (US); Teunissen, Paula Johanna Maria, Palo Alto, California 94304 (US); Van Brussel-Zwijnen, Marco, Palo Alto, CA 94304 (US); Scheffers, Martijn, Palo Alto, California 94304 (US); Guijt, Kees-jan, Palo Alto, California 94304 (US); Zou, Zhengzheng, Palo Alto, California 94304 (US); Tang, Zhongmei, Palo Alto, California 94304 (US); Qian, Zhen, Palo Alto, California 94304 (US); Ge, Jing, Palo Alto, California 94304 (US); Zhang, Zhenghong, Palo Alto, California 94304 (US)
(72) Inventor: KOOPS, Bart C., Palo Alto, California 94304 (US); TEUNISSEN, Paula Johanna Maira, Palo Alto, California 94304 (US); VAN BRUSSEL-ZWIJNEN, Marco, Palo Alto, California 94304 (US); SCHEFFERS, Martijn, Palo Alto, California 94304 (US); GUIJT, Kees-Jan, Palo Alto, California 94304 (US); ZOU, Zhengzheng, Palo Alto, California 94304 (US); TANG, Zhongmei, Palo Alto, California 94304 (US); QIAN, Zhen, Palo Alto, California 94304 (US); GE, Jing, Palo Alto, California 94304 (US); ZHANG, Zhenghong, Palo Alto, California 94304 (US)
(74) Representative: Mewburn Ellis LLP
(86) International application number: PCT/US2016/067685
(87) International publication number: WO 2017/112635

(56) References cited:
- WO-A1-2014/028436
- WO-A1-2014/058572
- WO-A1-2014/092960
- WO-A1-2014/093123

## Description

### CROSS-REFERENCE TO RELATED APPLICATIONS

### TECHNICAL FIELD

The present methods and compositions relate to granular starch-converting glucoamylases and α-amylases. The enzymes can be used to perform enzymatic starch hydrolysis of granular starch at or below the gelatinization temperature of insoluble granular starch.

### BACKGROUND

The conversion of insoluble granular starch to glucose or other soluble saccharides like-dextrins is often part of important large-scale processes to obtain end-products, such as sugar sweeteners, specialty syrups, enzymes, proteins, alcohol (*e.g.*, ethanol, butanol), organic acids (lactic acid, succinic acid, citric acid) and specialty biochemicals such as amino acids, (lysine, monosodium glutamate) and 1-3 propanediol. The partial crystalline nature of starch granules imparts insolubility in cold water. Solubilization of starch granules in water requires a tremendous amount of heat energy to disrupt the crystalline structure. The more water used to solubilize the granules, the more energy is required to heat the water. More energy is also required if evaporation of water from the end-product is required.

Solubilization of starch in a starch-water mixture can be performed by direct or indirect heating systems, such as direct heating by steam injection (see, for example, Starch Chemistry and Technology, eds R. L. Whistler et al., 2nd Ed., 1984 Academic Press Inc., Orlando, FL and Starch Conversion Technology, Eds. G.M.A. Van Beynum et al., Food Science and Technology Series, Marcel Dekker Inc., NY). A typical conventional starch liquefaction system delivers an aqueous starch slurry under high pressure to a direct steam injection cooker that raises the slurry temperature from about 35-40°C to 107-110°C. The slurry generally contains a thermal-stable alpha amylase in which case the pH is adjusted to favor the alpha amylase. Granular starch slurry resulting from wet milling usually has a dry solid content of 40 to 42%. The concentration is generally diluted to 32% to 35% dry solids before heating above the gelatinization temperature. Without this dilution the viscosity during the high temperature jet-cooking process would be likely so high that unit operation system cannot handle the slurry.

An alternative to the above conventional process has been described in which problems of excessive viscosity are avoided by not heating the granular starch slurry above the gelatinization temperature (see, e.g., US 7,618,795 and US 20050136525). Instead, the granular starch is solubilized by enzymatic hydrolysis below the gelatinization temperature. Such "low-temperature" systems (known also as "no-cook" or "cold-cook") have been reported to be able to process higher concentrations of dry solids than conventional systems (e.g., up to 45%). However, no-cook systems have the disadvantage that a relatively long incubation of about 24 hours or more at moderately elevated temperature is required for substantially complete solubilization. The longer incubation is itself associated with high energy costs.

Because of the large scale on which granular starch is processed, even seemingly small improvements in efficiency can have great economic advantage. However, the conversion process has already been extensively analyzed to identify and implement such improvements (see, *e.g.*, Martin & Brumm at pp. 45- 77 in "Starch Hydrolysis Products: Worldwide Technology, production and applications New York, VCH Publishers, Inc. 1992 and Luenser, Dev. in Ind. Microbiol.24.79-96 (1993)).

### SUMMARY

The invention is defined in the appended claims.

The present methods and compositions relate to granular starch-converting glucoamylases and α-amylases. The enzymes can be used to perform enzymatic starch hydrolysis of granular starch at or below the gelatinization temperature of insoluble granular starch: In one aspect, a method for processing granular starch is provided, according to claim 1.

WO 2014/093123 A1 describes a fungal alpha-amylase from *Aspergillus fumigatus* (AfAmyl), which may be used in combination with a glucoamylase and a pullulanase in a saccharification reaction. WO 2014/058572 A1 describes a fungal alpha-amylase from *Talaromyces emersonii* (TeAmyl), along with variants of the same, which may be used in combination with a glucoamylase in a saccharification reaction. WO 2014/028436 AI1 describes a fungal alpha-amylase from *Aspergillus clavatus* (AcAmyl), which may be used with a glucoamylase and an isoamylase in a saccharification reaction. WO 2014/092960 A1 describes fungal glucoamylases from *Aspergillus fumigatus* expressed in *Trichoderma reesei* host cells (AfGATR).

In some embodiments, the increased total glucose equivalents is at least 5% higher, and preferably at least 10% higher, compared to the amount produced by contacting the same slurry with the glucoamylase and α-amylase from *Aspergillus kawachii* (AkAA) having the amino acid sequence of SEQ ID NO: 2.

6. In some embodiments of the method of any of the preceding paragraphs, the method results in the production of glucose, maltose, oligosaccharides, or a mixture thereof, optionally in the form of a syrup.

7. In some embodiments, the method of any of the preceding paragraphs further comprising contacting the saccharides with a fermenting organism to produce an end of fermentation product; wherein the contacting results in increased production of an end of fermentation product compared to contacting the same slurry with the glucoamylase and α-amylase from *Aspergillus kawachii* (AkAA) having the amino acid sequence of SEQ ID NO: 2.

8. In some embodiments, the end of fermentation product is ethanol.

9. In some embodiments, the end of fermentation product is a non-ethanol biochemical.

In some embodiments, the glucoamylase and the granular starch-converting α-amylase are added simultaneously.

In some embodiments, the glucoamylase and/or the granular starch-converting α-amylase and the fermenting organism are added simultaneously.

In some embodiments, the glucoamylase and/or the granular starch-converting α-amylase are produced by a fermenting organism.

In some embodiments, the method of any of the preceding paragraphs further comprising the addition of an additional enzyme to the slurry.

In another aspect, a composition comprising a granular starch-converting α-according to claim 10. amylase in combination with a glucoamylase is provided!

22. In another aspect, a fermenting organism capable of producing a granular starch-converting α-amylase and a glucoamylase, according to claim 11.

These and other aspects and embodiments of the compositions and methods will be apparent from the present description.

### DETAILED DESCRIPTION

### Definitions

Prior to describing the compositions and methods in detail, the following terms and abbreviations are defined.

Unless otherwise defined, all technical and scientific terms used have their ordinary meaning in the relevant scientific field. Singleton, et al., Dictionary of Microbiology and Molecular Biology, 2d Ed., John Wiley and Sons, New York (1994), and Hale & Markham, Harper Collins Dictionary of Biology, Harper Perennial, NY (1991) provide the ordinary meaning of many of the terms describing the invention.

"Starch" refers a polysaccharide composed of glucose units that occurs widely in plant tissues in the form of storage granules, consisting of amylose and amylopectin. with the formula (C6H10O5)x, with X being any number. In particular, the term refers to any plant-based material, such as for example, grains, cereals, grasses, tubers and roots and more specifically wheat, barley, corn, rye, rice, sorghum, legumes, cassava, millet, potato, sweet potato, and tapioca.

"Granular starch" refers to uncooked (raw) starch, which has not been subject to gelatinization.

The term "granular starch-converting glucoamylase" refers to a glucoamylase that has increased activity on granular starch compared to the glucoamylase from Trichoderma reesei (TrGA) having the amino acid sequence of SEQ ID NO: 1, using the assays described in the Examples.

The term "granular starch-converting α-amylase" refers to an α-amylase that has increased activity on granular starch compared to the α-amylase from Aspergillus kawachii (AkAA) having the amino acid sequence of SEQ ID NO: 2, using the assays described in the Examples.

The terms "same glucoamylase" and "same α-amylase" with reference to an enzyme used for comparison purposes, refer to the identical enzyme (based on amino acid sequence) at the equivalent concentration and specific activity, such that the effect of other changes in the conditions can be experimentally evaluated.

"Starch gelatinization" means solubilization of starch molecules to form a viscous suspension.

"Gelatinization temperature" is the lowest temperature at which gelatinization of a starch containing substrate begins. The exact temperature of gelatinization depends on the specific starch and may vary depending on factors such as plant species and environmental and growth conditions. The initial starch gelatinization temperature ranges for a number of granular starches which may be used in accordance with the processes herein include barley (52-59° C), wheat (58-64° C), rye (57-70° C), corn (62-72° C), high amylose corn (67-80° C), rice (68-77° C), sorghum (68-77° C), potato (58- 68° C), tapioca (59-69° C) and sweet potato (58-72° C) (Swinkels, pg. 32-38 in STARCH CONVERSION TECHNOLOGY, Eds Van Beynum et al., (1985) Marcel Dekker Inc. New York and The Alcohol Textbook 3.sup.rd ED. A Reference for the Beverage, Fuel and Industrial Alcohol Industries, Eds Jacques et al., (1999) Nottingham University Press, UK). Gelatinization involves melting of crystalline areas, hydration of molecules and irreversible swelling of granules. The gelatinization temperature occurs in a range for a given grain because crystalline regions vary in size and/or degree of molecular order or crystalline perfection. STARCH HYDROLYSIS PRODUCTS Worldwide Technology, Production, and Applications (eds/ Shenck and Hebeda, VCH Publishers, Inc, New York, 1992) at p. 26.

"DE" or "dextrose equivalent" is an industry standard for the concentration of total reducing sugars, and is expressed as % D-glucose on a dry weight basis. Unhydrolyzed granular starch has a DE that is essentially 0 and D-glucose has a DE of 100.

"Glucose syrup" refers to an aqueous composition containing glucose solids. Glucose syrup has a DE of more than 20. Some glucose syrup contain no more than 21% water and no less than 25% reducing sugar calculated as dextrose. Some glucose syrups include at least 90% D-glucose or at least 95% D-glucose. Sometimes the terms glucose and glucose syrup are used interchangeably.

"Hydrolysis of starch" is the cleavage of glucosidic bonds in starch with the addition of water molecules.

A "slurry" is an aqueous mixture containing insoluble starch granules in water.

The term "total sugar content" refers to the total soluble sugar content present in a starch composition including monosaccharides, oligosaccharides and polysaccharides.

The term "dry solids" (ds) refer to dry solids dissolved in water, dry solids dispersed in water or a combination of both. Dry solids thus include granular starch, and its hydrolysis products, including glucose.

"Dry solid content" refers to the percentage of dry solids both dissolved and dispersed as a percentage by weight with respect to the water in which the dry solids are dispersed and/or dissolved. The initial dry solid content of starch is the weight of granular starch corrected for moisture content over the weight of granular starch plus weight of water. Subsequent dry solid content can be determined from the initial content adjusted for any water added or lost and for chemical gain. Subsequent dissolved dry solid content can be measured from refractive index as indicated below.

The term "high DS" refers to aqueous starch slurry with a dry solid content greater than 38% (wt/wt).

"Dry substance starch" refers to the dry starch content of a substrate, such as a starch slurry, and can be determined by subtracting from the mass of the subtrate any contribution of non-starch components such as protein, fiber, and water. For example, if a granular starch slurry has a water content of 20% (wt/wt)., and a protein content of 1% (wt/wt), then 100 kg of granular starch has a dry starch content of 79 kg. Dry substance starch can be used in determining how many units of enzymes to use.

"Refractive Index Dry Substance" (RIDS) is the determination of the refractive index of a starch solution at a known DE at a controlled temperature then converting the RI to dry substance using an appropriate relationship, such as the Critical Data Tables of the Corn Refiners Association

"Degree of polymerization (DP)" refers to the number (n) of anhydroglucopyranose units in a given saccharide. Examples of DP1 are the monosaccharides, such as glucose and fructose. Examples of DP2 are the disaccharides, such as maltose and sucrose. A DP4+ (>DP3) denotes polymers with a degree of polymerization of greater than 3.

The term "contacting" refers to the placing of referenced components (including but not limited to enzymes, substrates, and fermenting organisms) in sufficiently close proximity to affect an expect result, such as the enzyme acting on the substrate or the fermenting organism fermenting a substrate. Those skilled in the art will recognize that mixing solutions can bring about "contacting."

The term "fermenting organism" refers to any organism, including bacterial and fungal (including filamentous fungi and yeast), suitable for producing a desired end of fermentation (EOF) product.

The term "end of fermentation (EOF) product," or simply "fermentation product," is any carbon-source derived molecule product that is produced by a fermenting organism, i.e., an organism capable of fermenting fermentable sugars and includes, but is not limited to, metabolites, such as citric acid, lactic acid, succinic acid, acetic acid, monosodium glutamate, gluconic acid, sodium gluconate, calcium gluconate, potassium gluconate, itaconic acid and other carboxylic acids, glucono delta-lactone, sodium erythorbate, glutamic acid, tryptophan, threonine, methionine, lysine and other amino acids, omega-3 fatty acid, isoprene, 1,3-propanediol, ethanol, methanol, propanol, butanol, other alcohols, and other biochemicals and biomaterials.

"Enzyme activity" refers to the action of an enzyme on its substrate.

An "α-amylase (E.C. class 3.2.1.1)" is an enzyme that catalyze the hydrolysis of alpha-1,4-glucosidic linkages. These enzymes have also been described as those catalysing the exo- or endohydrolysis of 1, 4-α-D-glucosidic linkages in polysaccharides containing 1, 4-α-linked D-glucose units. Another term used to describe these enzymes is glycogenase. Exemplary enzymes include alpha-1,4-glucan 4-glucanohydrase glucanohydrolase.

A "glucoamylase" refers to an amyloglucosidase class of enzymes (EC.3.2.1.3, glucoamylase, alpha-1, 4-D-glucan glucohydrolase) are enzymes that remove successive glucose units from the non-reducing ends of starch. The enzyme can hydrolyze both linear and branched glucosidic linkages of starch, amylose and amylopectin. The enzymes also hydrolyze alpha-1, 6 and alpha -1, 3 linkages although at much slower rates than alpha-1, 4 linkages.

"Pullulanase" also called debranching enzyme (E.C. 3.2.1.41, pullulan 6-glucanohydrolase), is capable of hydrolyzing alpha 1-6 glucosidic linkages in an amylopectin molecule.

"Yield" refers to the amount of a desired end-product/products (e.g., glucose) as a percentage by dry weight of the starting granular starch.

The phrase "simultaneous saccharification and fermentation (SSF)" refers to a process in the production of end of fermentation products in which a microbial organism, such as an ethanologenic microorganism, and at least one enzyme, such as one or more glucoamylase, are present during the same process step. SSF includes the contemporaneous hydrolysis of starch substrates (granular, liquefied, or solubilized) to saccharides, including glucose, and the fermentation of the saccharides into alcohol or other biochemical or biomaterial in the same reactor vessel.

Sequence identity can be determined by aligning sequences using algorithms, such as BESTFIT, FASTA, and TFASTA in the Wisconsin Genetics Software Package Release 7.0, Genetics Computer Group, 575 Science Dr., Madison, WI), using default gap parameters, or by inspection, and the best alignment (i.e., resulting in the highest percentage of sequence similarity over a comparison window). Percentage of sequence identity is calculated by comparing two optimally aligned sequences over the length of the shorter sequence (if lengths are unequal), determining the number of positions at which the identical residues occurs in both sequences to yield the number of matched positions, dividing the number of matched positions by the total number of matched and mismatched positions not counting gaps, and multiplying the result by 100 to yield the percentage of sequence identity. Unless otherwise specified, percent amino acid sequence identity as used herein is calculated using the CLUSTAL W algorithm with default parameters. See Thompson et al. (1994) Nucleic Acids Res. 22:4673-4680. Default parameters for the CLUSTAL W algorithm are:

| | |
|---|---|
| Gap opening penalty: | 10.0 |
| Gap extension penalty: | 0.05 |
| Protein weight matrix: | BLOSUM series |
| DNA weight matrix: | IUB |
| Delay divergent sequences %: | 40 |
| Gap separation distance: | 8 |
| DNA transitions weight: | 0.50 |
| List hydrophilic residues: | GPSNDQEKR |
| Use negative matrix: | OFF |
| Toggle Residue specific penalties: | ON |
| Toggle hydrophilic penalties: | ON |
| Toggle end gap separation penalty | OFF. |

The term "comprising" and its cognates are used in their inclusive sense; that is, equivalent to the term "including" and its corresponding cognates.

Numeric ranges are inclusive of the numbers defining the range. Some preferred subranges are also listed, but in any case, reference to a range includes all subranges defined by integers included within a range.

The term "total glucose equivalent" refers to a manner to calculate starch conversion in a process, such as a fermentation process, so that the starch conversion in different processes can be compared. Comparing processes can be difficult because intermediate products and end products are formed next to side products. For example, in an ethanol fermentation process starch is converted into dextrins, which are converted into glucose and the glucose is fermented into ethanol by a yeast. The yeast is also converting glucose into glycerol as a main side product and bacteria present in the process can convert glucose while producing acetic acid and lactic acid. The glucose equivalent is a way in which all these soluble components, which can be measured by for example HPLC, are mathematically converted to glucose so they can be added up and form the glucose equivalent of all soluble components. For example, 1 mole a disaccharide like maltose, with a molar weight of 342.30 g/mol is converted into 2 moles glucose with a molar weight 180.02 g/mol. The mathematical conversion for maltose is then (2 x 180.02) / 342.30 = 1.052 and each gram / liter of maltose is multiplied with this 1.052 to convert into the glucose equivalent for maltose. People skilled in the art will be able to do this for the other major components in the fermentation process. For DPn an average degree of polymerization of 10 is chosen. This way the glucose equivalents for ethanol, glycerol, acetic acid, lactic acid, Succinic acid, DP1, DP2, DP3 and DPn are calculated and added to form the total glucose equivalents for the process. Since only soluble components are measured, a process in which a similar amount of starch is converted will show a similar "total glucose equivalent" value. If more starch is dissolved, an increase in total glucose equivalent is visible.

### Granular starch-converting glucoamylases and α-amylases

Low-temperature starch hydrolysis processes, also known as "no-cook" or "cold-cook" processes, have been described (see, e.g., US 7,618,795 and US 20050136525). In a cold cook process, granular starch is solubilized by enzymatic hydrolysis at or below the gelatinization temperature. Such low temperature processes represent an alternative to conventional starch hydrolysis with certain advantages, such as avoiding the high starch slurry viscosity created by heating granular starch above the gelatinization temperature and the high operational cost of such heating.

Because the cold-cook process does not require a jet cooker, it can be performed in ethanol production plants that were originally designed to use such feed stocks as sugar cane. This allows such production plants to utilize, for example, corn or sugar cane, depending on which is less expensive or more available at the time. Such plants may benefit from the use of a separation device to remove unfermentable corn material prior to introduction to the plant to avoid fouling equipment that was not designed to handle such material. Separation can be performed by centrifugation, filtration, or other conventional methods. The cost of installing a separation device is expected to be substantially less than installing a jet cooker

However, no-cook systems have the disadvantage that a relatively long incubation of about 24 hours or more at moderately elevated temperature is required for substantially complete solubilization. The longer incubation is itself associated with high energy costs and reduced throughput and the long incubation time at the moderately elevated temperature can lead to contamination.

The present compositions and methods are based on the observation that certain glucoamylases (GA) and α-amylases (AA) show a high degree of activity on granular starch. The observations are based on extensive empirical testing of a large number of GA and AA in raw starch hydrolysis assays using current commercial benchmarks as references. Because of the large number of enzymes tested, only GA and AA that performed better than benchmark enzymes, i.e., Trichoderma *reesei* glucoamylase (TrGA) (SEQ ID NO: 1) and *Aspergillus kawachii* α-amylase (AkAA) (SEQ ID NO: 2) are described, herein.

The amino acid sequences of TrGA and AkAA are shown, below:
SEQ ID NO: 1 (TrGA) from *Trichoderma reesei* 
SVDDFISTETPIALNNLLCNVGPDGCRAFGTSAGAVIASPSTIDPDYYYMWTRDSALV
FKNLIDRFTETYDAGLQRRIEQYITAQVTLQGLSNPSGSLADGSGLGEPKFELTLKPFT
GNWGRPQRDGPALRAIALIGYSKWLINNNYQSTVSNVIWPIVRNDLNYVAQYWNQT
GFDLWEEVNGSSFFTVANQHRALVEGATLAATLGQSGSAYSSVAPQVLCFLQRFWV
SSGGYVDSNINTNEGRTGKDVNSVLTSIHTFDPNLGCDAGTFQPCSDKALSNLKVVV
DSFRSIYGVNKGIPAGAAVAIGRYAEDVYYNGNPWYLATFAAAEQLYDAIYVWKKT
GSITVTATSLAFFQELVPGVTAGTYSSSSSTFTNIINAVSTYADGFLSEAAKYVPADGS
LAEQFDRNSGTPLSALHLTWSYASFLTATARRAGIVPPSWANSSASTIPSTCSGASVV
GSYSRPTATSFPPSQTPKPGVPSGTPYTPLPCATPTSVAVTFHELVSTQFGQTVKVAG
 
NAAALGNWSTSAAVALDAVNYADNHPLWIGTVNLEAGDVVEYKYINVGQDGSVT
WESDPNHTYTVPAVACVTQVVKEDTWQS
SEQ ID NO: 2 (AkAA) from *Aspergillus kawachii*

LSAAEWRTQSIYFLLTDRFGRTDNSTTATCNTGDQIYCGGSWQGIINHLDYIQGMGF
TAIWISPITEQLPQDTSDGEAYHGYWQQKIYNVNSNFGTADDLKSLSDALHARGMYL
MVDVVPNHMGYAGNGNDVDYSVFDPFDSSSYFHPYCLITDWDNLTMVQDCWEGD
TIVSLPDLNTTETAVRTIWYDWVADLVSNYSVDGLRIDSVEEVEPDFFPGYQEAAGV
YCVGEVDNGNPALDCPYQKYLDGVLNYPIYWQLLYAFESSSGSISNLYNMIKSVASD
CSDPTLLGNFIENHDNPRFASYTSDYSQAKNVLSYIFLSDGIPIVYAGEEQHYSGGDV
PYNREATWLSGYDTSAELYTWIATTNAIRKLAISADSDYITYANDPIYTDSNTIAMRK
GTSGSQIITVLSNKGSSGSSYTLTLSGSGYTSGTKLIEAYTCTSVTVDSNGDIPVPMAS
GLPRVLLPASVVDSSSLCGGSGNTTTTTTAATSTSKATTSSSSSSAAATTSSSCTATST
TLPITFEELVTTTYGEEVYLSGSISQLGEWDTSDAVKLSADDYTSSNPEWSVTVSLPV
GTTFEYKFIKVDEGGSVTWESDPNREYTVPECGSGSGETVVDTWR

The GA that performed better in combination with AkAA, or better in a blend with a different AA, are listed in the following table:

| **Name** | **Abbr.** | **Source organism** | **SEQ ID NO** |
|---|---|---|---|
| GA-1805 | AteGA1 | *Aspergillus terreus* | 3 |
| GA-2040 | AfuHT3 | *Aspergillus fumigatus* | 4 |
| GA-2331 | NfiGA1 | *Neosartorya fischeri* | 5 |
| GA-2437 | AfuGA2 | *Neosartorya fumigata* | 6 |
| GA-2439 | PmaGA1 | *Penicillium marneffei* | 7 |
| GA-2441 | TstGA2 | *Talaromyces stipitatus* | 8 |
| GA-2442 | MacGA1 | *Metarhizium acridum* | 9 |
| GA-2578 | ScoGA1 | *Schizophyllum commune* | 10 |
| GA-2722 | Tat GA2 | *Trichoderma atroviridis; Hypocrea atroviridis* | 11 |
| GA-3275 | BadGA1 | *Bjerkandera adusta* | 12 |
| GA-3280 | GspGA1 | *Ganoderma spp* | 13 |
| GA-3283 | TveGA3 | *Termetes versicolor* | 14 |
| GA-3294 | HsuGA3 | *Hypholoma sublateritium* | 15 |
| GA-3298 | FmeGA1 | *Fomitiporia mediterranea* | 16 |
| GA-3301 | PstGA2 | *Punctularia strigosozonata* | 17 |
| GA-3317 | PbrGA1 | *Phlebia brevispora Nakasone* | 18 |
| GA-4686 | SzeGA2 | *Sarocladium zeae* | 19 |
| GA-4688 | PoxGA5 | *Penicillium oxalicum* | 20 |

The amino acid sequences are shown, below:
SEQ ID NO: 3; GA-1805 (AteGA1) from *Aspergillus terreus* 
APQLAPRATTSLDAWLASETTVALDGILDNVGSSGAYAKSAKSGIVIASPSTSDPDYY
YTWTRDAALTVKALIDLFRNGETSLQTVIMEYISSQAYLQTVSNPSGSLSTGGLAEPK
YYVDETAYTGSWGRPQRDGPALRATAMIDFGNWLIDNGYSTYASSIVWPIVRNDLS
YVAQYWNQTGYDLWEEVNGSSFFTIAVQHRALVEGSTFASKVGASCSWCDSQAPQ
VLCFLQRFWTGSYIMANFGGGRSGKDANTVLGSIHTFDPNAGCDDTTFQPCSPRALA
NHKVYTDSFRSIYSINSGISSGKAVAVGRYPEDSYYNGNPWFLTTLAAAEQLYDAIY
QWQKIGSITITDVSLAFFKDLYSSAAVGTYASSSSAFTSIVSAVKTYADGYMSIVQTH
AMTNGSLSEQFGKSDGFSLSARDLTWSYAALLTANLRRNSVVPPSWGETTATSVPSV
CSATSATGTYSTATNTAWPSTLTSGTGATTTTSKATSSSTTTTSSASSTTVECVVPTA
VAVTFDEVATTTYGENVYVVGSISQLGSWDTSKAVALSASKYTSSNNLWYVTVTLP
AGTTFQYKFIRVSSSGSVTWESDPNRSYTVPSACGTSTAVVNTTWR
SEQ ID NO: 4; GA-2040 (AfuHT3) from *Aspergillus fumigatus* 
APQLSARATGSLDSWLGTETTVALNGILANIGADGAYAKSAKPGIIIASPSTSEPDYY
YTWTRDAALVTKVLVDLFRNGNLGLQKVITEYVNSQAYLQTVSNPSGGLASGGLAE
PKYNVDMTAFTGAWGRPQRDGPALRATALIDFGNWLIDNGYSSYAVNNIWPIVRND
LSYVSQYWSQSGFDLWEEVNSMSFFTVAVQHRALVEGSTFAKRVGASCSWCDSQA
PQILCYMQSFWTGSYINANTGGGRSGKDANTVLASIHTFDPEAGCDDTTFQPCSPRA
LANHKVYTDSFRSVYAINSGIPQGAAVSAGRYPEDVYYNGNPWFLTTLAAAEQLYD
AIYQWKKIGSISITSTSLAFFKDIYSSAAVGTYASSTSTFTDIINAVKTYADGYVSIVQA
HAMNNGSLSEQFDKSSGLSLSARDLTWSYAAFLTANMRRNGVVPAPWGAASANSV
PSSCSMGSATGTYSTATATSWPSTLTSGSPGSTTTVGTTTSTTSGTAAETACATPTAV
AVTFNEIATTTYGENVYIVGSISELGNWDTSKAVALSASKYTSSNNLWYVSVTLPAG
TTFEYKYIRKESDGSIVWESDPNRSYTVPAACGVSTATENDTWQ
SEQ ID NO: 5; GA-2331 (NfiGA1) from *Neosartorya fischeri* 
APQLSPRATGSLDSWLATESTVSLNGILANIGADGAYAKSAKPGIIIASPSTSDPDYYY
TWTRDAALVTKVLVDLFRNGNLGLQKVITEYVNSQAYLQTVSTPSGGLSSGGLAEP
KYNVDMTAFTGAWGRPQRDGPALRATALIDFGNWLIDNGYSSYAVNNIWPIVRNDL
SYVSQYWSQSGFDLWEEVNSMSFFTVAVQHRALVEGSTFAKRVGASCSWCDSQAP
QILCYMQSFWTGSYINANTGGGRSGKDANTVLASIHTFDPEAGCDDTTFQPCSPRAL
ANHKVYTDSFRSVYAINSGIPQGVAVSAGRYPEDVYYNGNPWFLTTLAAAEQLYDA
IYQWKKIGSISITSTSLAFFKDIYSSVAVGTYASSSSTFTAIIDAVKTYADGYVSIVEAH
AMTNGSLSEQFDKSSGMSLSARDLTWSYAALLTANMRRNGVVPAPWGAASANSVP
SSCSMGSATGTYSTATATSWPSTLTSGSPSDTTSGTTPGTTTTTSACTTPTSVAVTFDE
IATTTYGENVYIIGSISQLGSWDTSKAVPLSSSKYTSSNNLWYVTINLPAGTTFEYKYI
RKESDGSIEWESDPNRSYTVPSACGVSTATEKDTWR
SEQ ID NO: 6; GA-2437 (AfuGA2) from *Neosartorya fumigata* 
APQLSARATGSLDSWLGTETTVALNGILANIGADGAYAKSAKPGIIIASPSTSEPDYY
YTWTRDAALVTKVLVDLFRNGNLGLQKVITEYVNSQAYLQTVSNPSGGLASGGLAE
PKYNVDMTAFTGAWGRPQRDGPALRATALIDFGNWLIDNGYSSYAVNNIWPIVRND
LSYVSQYWSQSGFDLWEEVNSMSFFTVAVQHRALVEGSTFAKRVGASCSWCDSQA
PQILCYMQSFWTGSYINANTGGGRSGKDANTVLASIHTFDPEAGCDDTTFQPCSPRA
LANHKVYTDSFRSVYAINSGIPQGAAVSAGRYPEDVYYNGNPWFLTTLAAAEQLYD
 
AIYQWKKIGSISITSTSLAFFKDIYSSAAVGTYASSTSTFTDIINAVKTYADGYVSIVQA
HAMNNGSLSEQFDKSSGLSLSARDLTWSYAAFLTANMRRNGVVPAPWGAASANSV
PSSCSMGSATGTYSTATATSWPSTLTSGSPGSTTTVGTTTSTTSGTATETACATPTAV
AVTFNEIATTTYGENVYIVGSISELGNWDTSKAVALSASKYTSSNNLWYVSVTLPAG
TTFEYKYIRKESDGSIVWESDPNRSYTVPAACGVSTATENDTWR
SEQ ID NO: 7; GA-2439 (PmaGA1) from *Penicillium marneffei* 
APQFSPRATVGLDAWLASETTFSLNGILANIGSSGAYSASAKPGVVIASPSTNNPNYY
YTWTRDSALTLKVLIDLFGNGNLSLQTVIEEYINAQAYLQTVSNPSGDLSSGAGLAEP
KYNVDMSPFTGGWGRPQRDGPALRAIALIEFGNWLIDNGYSSYAVNNIWPIVRNDLS
YVSQYWSQSGFDLWEEVNSMSFFTVANQHRALVQGSTFAARVGASCSWCDSQAPQ
ILCYMQTFWTGSYINANTGGGRSGKDSNTVLTTIHTFDPEATCDDVTFQPCSPRALA
NHKVYTDSFRSIYGVNSGIAQGVAVSVGRYPEDSYYGGNPWFLSNLAAAEQLYDAI
YQWNKIGSITITSTSLAFFKDVYSSAAVGTYASGSTAFTSIISAVKTYADGYVSIVQGH
AAANGSLSEQFDRNSGVEISARDLTWSYAALLTANLRRNGVMPPSWGAASANSVPS
SCSMGSATGTYSTPTATAWPSTLTSATGIPVTTSATASVTKATSATSTTTSATTCTTPT
SVAVTFDEIATTTYGENVFIVGSISQLGSWDTSKAIALSASQYTSSNHLWFATLSLPAG
TTFQYKYIRKESNGSIVWESDPNRSYTVPSGCGVSTATENDTWR
SEQ ID NO: 8; GA-2441 (TstGA2) from *Talaromyces stipitatus* 
APGLSPRASTSLDAWLATETTVSLSGILANIGADGAYSKSAKPGVVIASPSTDNPNYY
YTWTRDSALTLKVLIDLFRNGNLGLQTVIEEYVNAQAYLQTVSNPSGDLSSGAGLAE
PKFNVDMSAFTGSWGRPQRDGPALRAIALIDFGNWLIENGYTSLAANNIWPIVRNDL
SYVAQYWSQSGFDLWEEVNSMSFFTVANQHRSLVEGSTFAAKVGASCSWCDSQAP
QILCYMQTFWTGSYMNANTGGGRSGKDANTVLTSIATFDPEATCDDVTFQPCSPRA
LANHKVYTDSFRSVYGLNSGIAEGVAVAVGRYPEDSYYNGNPWFLSNLAAAEQLY
DAIYQWNKIGSITITSTSLAFFKDVYSSAAVGTYASGSSAFTSIINAVKTYADGYISVV
QSHAMNNGSLSEQFDKNTGAELSARDLTWSYAALLTANMRRNGVVPPSWGAASAT
SIPSSCTTGSAIGTYSTPTATSWPSTLTSGTGSPGSTTSATGSVSTSVSATTTSAGSCTT
PTSVAVTFDEIATTSYGENVYIVGSISQLGSWNTANAIALSASKYTTSNNLWYVTINL
PAGTTFQYKYIRKESDGTVKWESDPNRSYTVPSACGVSTATENDTWR
SEQ ID NO: 9; GA-2442 (MacGA1) from *Metarhizium acridum* 
HRDDLHGFITKQKSISLHGVLANIGSDGSRAQGAAAGAVVASPSKSDPDYWYTWSR
DSALTFKVLIELFIGGKKSLQPKIEQYITAQAHLQGVSNPSGGPDTGGLGEPKFHVNL
TAFTGSWGRPQRDGPPLRATALTIYANWLIANGGQAEAANTVWPIIAKDLSYTVQY
WNRTGFDLWEEINGSSFFTLSASFRALVEGATLAKALGKQCPDCETNAPRILCFLQSF
WANGYIDSNINVNDGRTGKDVNSIISSIHTFDPAAACTDATFQPCSSRALANHKAVV
DSFRTIYTVNKGRRPGRAAAVGRYSEDVYYNGNPWYLATMAAAEQMYAAVYQWR
EIGSITVDATSLPFFSDLIPNIAAGTYAKNSATFTSIIKAATAYGDDFVRVVKQYTPAD
GSLAEQYDRETGSPKSAVHLTWSYASFVGAVERRSGIVPPSWGEPNSNTVPKVCEAP
PSCDSTMTFNVKVTTVPGESIYVVGSITELKNWSPADAVPLDASQYTPSNPLWSAKV
TIPAGTNFEYKYIKKTSDGTVVWESDPNRSATSSTGCQSNGTLNDQWR
SEQ ID NO: 10; GA-2578 (ScoGA1) from *Schizophyllum commune* 
QTSAADAYVSAESPIAQAGILANIGPSGSKSHGAASGVIIASPSTSNPDYLYTWTRDA
ALVSRALVDEFIEGESSLQSVIDSYVSSQQKLQRVDNPSGSYTSGGLGEPKFNIDLTAF
TGAWGRPQRDGPALRAITLITYGNHLLSSGNTSYVTDTIWPVVKADLDYVVSYWNQ
TGFDLWEEVSSSSFFTTAEQHTALRLGATFATAVGASASTYLTQADNVLCFLQSYWN
SNGGYATANTGGGRSGIDANTVLTSIHTFDIEAGCDSVTFQPCSDRALSNLKVYVDSF
RGLYSINPTGATDPILTGRYKEDVYYNGNPWYLTTFAVAEQLYDALNTWDKLGSLD
VTSTSLAFFKQFDSSITAGTYASSTSEYATLTSAIRNWADGFLEVLADFTPADGGLTE
QIDKSSGNPTSAADLTWSYASAITAFKARGGAIPASWGAAGLTVPATCSTGGGGGSG
GDTVAVTLNVQATTVYGENIYVTGSVNQLANWSPDNAIALNADNYPTWSVTVNLP
ANTQIEYKYIRKNNGQVTWESDPNRSITTSASGSFTQNDTWR
SEQ ID NO: 11; GA-2722 Tat GA2) from *Trichoderma atroviridis* / *Hypocrea atroviridis* 
VPRLRESRHEFDIVKRSASSFLETEVPIALADLLCNIGSAGSCAAGANSGIVIASPSKTN
PDYFYTWTRDSALVFKCIVDTFVNSYSASLQTEIENYINAQIVQGISNPSGSLSNSGT
GLGEPKFNVDETAFTGAWGRPQRDGPALRAIALITYSKWLINNGYQSTANSIVWPIIQ
NDISYVAQYWNQTGFDLWEEVNGSSFFTVANQHRALVEASALATSLGKSLPNASSQ
AAQALCFLQSFWSSSQGYIVANINQNNGRSGKDANTLLGSIHTFDPEGNCDASTFQP
CSDRTLANHKVVVDSFRSIYTINNGIPAGTAAAVGRYPEDSYQGGNPWYLNTLAAA
ELLYDALYQWKRIGAITVTSTSLAFFKDLDSSITVGTYSSSSSTYTTLYNAVSNYADG
FVNNVATYAPSNGSLAEQYNRNNGQPLSAYDLTWSYAALLTAAARRSGVVPYSWG
ETSASSVPSVCSYTSAVGSYSSASTGSWPPNQTPTDGSGSTTSKSTSVTVSSTSTSASS
TAVATSPVTVTFDEIVTTIFGQTIKIAGNVPVLGNWNTNNAVALSADGYTSSNHLWN
VGISFAPGTVIQYKYINVASNGDVTWEADPNHTYTVPATGATAVTVNNSWQS
SEQ ID NO: 12; GA-3275 (BadGA1) from *Bjerkandera adusta* 
QSSTVDAFIASESPIARTGLLANIGADGSKASGAKSGIVIASPSKSNPDYFYTWTRDAA
LVFKAIIDRYTSGEDTATRRQIDEYVSGQALLQQVSNPSGTVSTGGLAEPKYNVDMS
AFTGGWGRPQRDGPALRATAIIAYANWLVANGNTSYVTSTLWPVLKLDLDYVRDN
WNQTGFDLWEEINSSSFFTTAVQHRALREGNALAAKIGQTVSGYTTQADNVLCFLQS
YWNPSGGFATSNTGGGRSGKDANSVLTSIHTFDAAAGCDALTFQPCSDRALSNHKV
YVDSFRSIYSVNSGIASNAAVATGRYPEDSYYGGNPWYLTTLAAAEQLYDALTVWD
AQGSLNVTSVSLAFFQQFAPTVTAGTYPASSATYGTLTAAIRAYADGFVAVVAKYTP
SNGGLAEQYTRAGGTPTSAADLTWSYAAALTAFSAREGFTPASWGAKGLTAPAACN
TNSGGGSGGGSGNTVAVTFNVQATTVWGENIYLTGSVDALQNWSPDNALLLSSAN
YPTWSITVNLPPSTAIQYKYIRKNNGAVTWESDPNMSITTPGSGSATLSDTWR
SEQ ID NO: 13; GA-3280 (GspGA1) from *Ganoderma* spp. 
QSSADAYVASEASIAKAGLLANIGANGSKSEGAKAGIVVASPSTSNPDYLYTWTRDS
SLVFKTVIDQFTTGEDTSLRGLIDEFTAAQSILQQTSNPSGSVSTGGLGEPKFNVDETA
FTGAWGRPQRDGPALRATAIITYANWLLANGNGTSYVQNTLWPIIKLDLDYVENNW
NQSTFDLWEEVNSSSFFTTAVQHRALREGVALASAIGQTSVVSGYSAQADNLLCFLQ
SYWNSGSGFVTANTGGGRSGRDANTVLTSIHTFDVEAGCDAVTFQPCSDKALSNLK
VYVDAFRSIYGINSGIASNAAVATGRYPEDSYYNGNPWYLAVFAVAEQLYDALITW
DELGSLNVTSTSLAFFQQFDSSVTAGTYDSSSSTYSTLTSGIKGFADGFLEVNSKYTPS
TGALSEQFDKSSGSQLSASDLTWSYAAALTAFAARSGKTYASWGAAGLITTCGGSG
 
GGGGGSGTVSVTFNVQATTVFGENIYITGSVDALQNWSPDNALILSAANYPIWSITVS
LPASTVIEYKYIRKFNGQVTWESDPNDSITTPASGSYIENDTWR
SEQ ID NO: 14; GA-3283 (TveGA3) *Termetes versicolor* 
QSSVADAYVASESSIAKAGVLANIGPSGSKSQGAKAGIVVASPSTTNPDYLFTWTRD
TSLVFKALIDQLTSGEDPSLRGLVDMFTSSQAALQQVSNPSGTVSTGGLGEPKFNIDE
SAFTGAWGRPQRDGPALRSTAFISYANWLLDNGNTTYVTQTLWPVIKLDLDYVEAN
WNQTGFDLWEEVNSSSFFTTAVQHRALREGAAFATRIGQTSVVSGYTTQAANVLCF
LQSYWNPSGGFVTANTGGGRSGRDANTVLTSIHTFDPAAGCDATTFQPCSDKALSNL
KVYVDAFRSIYTINSGIAANAAVATGRYPEDSYQGGNPWYLATIAVAEQLYDALIV
WDQLGSINVTTTSLPFFQQFSSTVTTGTFASTSATYTTLTTAVRNFADGFIAVNAQFT
PSNGGLAEQFSRSNGQPVSAVDLTWSYAATLTAFHARAGLTYPGWGAAGLTVPAV
CSTSGSGSGGGGAGTVAVTFNVQATTFFGENIYITGSVDALQNWSPDNALLLSSANY
PIWSITVNLPASTSVQYKFIRKAPGELIWESDPNNQITTPASGTFTQSDTFR
SEQ ID NO: 15; GA-3294 (HsuGA3) from *Hypholoma sublateritium* 
QSSAVSSYLATESVIAKAGLLANIGPSGSKASGAVSGVVVAAPSTNPDYIFTWTRDSA
LVFKAIIDSFARGEDATLRTSIDQYVAAQKIQQQVSNPSGTVSSGGLGEPKFNVDLSA
FTGAWGRPQRDGPALRATALISYGNSLISASNTSYVLANIWPLVKLDLDYVAANWN
QTGFDLWEEVNSSSFFTTAVQHRALRQGAAFATALGQTASVAGYTTQAANVLCFLQ
SYWNPSQGYITANTGGGRSGKDANTALASIHTFDPTAGCDAATFQPCSDKALSSLKV
YVDSFRSIYTVNSAVASPGAVATGRYPEDSYFGGNPWYLATMAVAEQLYDALIVWK
AQGSLNVTSTSLAFFQQFSSAVTVGTYASTTATFTTLTTAIANQADGFVAIVQEFTPS
TGSLSEQYSRSNGAQLSANDLTWSYASILTAVTARNGLAGDNWGAAGLVVPSSCST
SGTGSSSGGGSSGTVAVTFKVTATTTFGENIYLTGSDDALEDWSPTSTLILSAATYPV
WSITVNLPASTALQYKYIRIFNGVTTWESDPNNAFTTVASGTQTLTDTWR
SEQ ID NO: 16; GA-3298 (FmeGA1) from *Fomitiporia mediterranea* 
QTAVDSYVATESPIAKTNLLANIGASGSKSQGAKPGIVIASPSTTNPNYLFTWTRDSSL
VFKTIIDQYTNGQDTSLRTLIDEFVSAEATLQQVSNPSGTVSTGGLGEPKFNIDETAFT
GAWGRPQRDGPALRATAIINYANYLLANDNSSFVTNTLWPILQLDLDYVAQDWNQT
TFDLWEEVDSTSFFTAAVQHRSLREGATLATKIGQTSVVSGYTTQAENILCFMQSFW
NAGGNFMTANTGGGRSGKDANTVLASIHTFDSSAGCDAATFQPCSDRALANLKTYV
DAFRSIYSINSGIASNAAVATGRYPEDVYFNGNPWYLTTLSVAEQLYDAITVWNAQG
SLNVTSVSQPFFALFQSDIAVGTYASSSSTFTSLLSSIKSFADGFVSVVAKYTPSNGGL
SEQYSKSDGTPTSAVDLTWSYAAALTAFAARDGFVPASWGAAGLTVPSTCSTSGSG
PGSGGTVAVTFNVQATTVFGENIYITGSVDALQNWSPDNAIILSAANYPTWSVTINLP
ASTTIQYKYIRKFNGAVTWESDPNMQITTPSGGTFIENDVWR
SEQ ID NO: 17; GA-3301 (PstGA2) from *Punctularia strigosozonata* 
QTASAAAYATTEAPIAKAGVLANIGPSGSKSQGAKAGIVIASPSTSNPDYLYTWTRDS
SLVFKALIDQYTSGIDTTLRGAIDNFFNAEKILQQVSNPSGTVSTGGLGEPKFNIDETA
FTGAWGRPQRDGPALRATALITYANYLYSTGNTTFVSNTLWPVIKLDLDYAANNWN
QTTFDLWEEVSSSSFFTTAVQHRSLREGATLATKLGVTSSASTYTSAASSLLCFLQSY
WNPAGGYITANTGGGRSGKDANTVLTSIHTFDPAAGCDAVTFQPCSDKALSNLKVY
VDSFRSIYGINSGIASNAAVATGRYPEDTYYNGNPWYLTTLAVAEQLYDALIVWNAR
 
GSLNVTSTSLAFFQQFSSSVTTGTYPSTSTTFTTLTSAVKTFADGFVAVVAKYTPSSG
ALSEQFDKSSGSQLSAADLTWSYAAALTAFEARNGTTFASWGAAGLTTSCSSSGSGS
GGGSGSSGSVPVNFQETATTVYGENIFIVGSISPLGNWDPNSAIALSAANYPNWQVSI
SLPASTTFQYKYIRKYNGAVTWESDPNRSFTTPSSGSYNENDTWR
SEQ ID NO: 18; GA-3317 (PbrGA1) from *Phlebia brevispora* Nakasone 
QTNVNSYVASESAFAKAGLLANIGPSGSKSSGADPGIVIASPSTTNPDYLYTWVRDSS
LVFKVLIDQYTTGVDTSLRTLIDEFVSAEAILQQVTNPSGSVTTGGLGEPKFNIDETAF
TGSWGRPQRDGPALRSTAIITYANWLLDNGNTSYVTETLWPVLELDLNYVMNNWN
QSTYDLWEEIDSSSFWTTAVQHRALRQGSALATRIGQTSMVSGYNTQAANVLCFLQ
SFWNPSGNYVTANTGGGRSGIDANTVLTSIHTFDPSAGCDATTFQPCSDKALANLKV
YTDSFRSIYSVNTGIASNAAVATGRYPEDVYMGGNPWYLATMAAAEQLYDALSVW
ESQGSLTVTPTSLAFFQMFDSGVQAGTYASSSSTFSSLTSAIQSLADGFVAIHAEYTPS
DGSLSEQFSRSNGSPTSAADLTWSYAAALTGFAARNGTQVASWGAAGLTVPATCQG
SPGPTVSVTFNVDATTVWGENIYITGSVDALENWSTTTALLLSSANYPIWSITVSLPA
NTNIQYKYIRIDNGAVTWESDPNNSLTTPASGSYTVNDTWR
SEQ ID NO: 19; GA-4686 (SzeGA2) from *Sarocladium zeae* 
RPGPAKVQLSTRAVGDFINSETPIALEQLLCNIGANGCNSAGVSSGLVIASPSKQDPD
YWYTWTRDSALVFKSIVDRFTNSYDAGLQRHITDYIVAQARLQGVSNPSGGFSDGS
GLAEPKYNVDGSAFTGAWGRPQRDGPALRAIAIMSYGEWLLDNSYTDTAKNIVWPV
VRNDLEYVAQYWNQTGFDLWEEVRGSSFFTIASQHQALVQGYRFAARVGASGAHY
QATAPSVLCFLQSFWNPSKGYIDSNINVNDGRTGLDANSILASIHTFDASIGCDSTTFQ
PCSDKALSNLKAVVDSFRFYNINNGIPKGTALAVGRYAEDVYYNGNPWYLNTLAAA
EQLYDAVYVWKQQGSVTVTATSRAFFADLIPNIAVGTYQSGSSTYNSIIQAVSQYGD
GFVNVVATYAQSNGSLAEQFSKQDGTPLSARDLTWSYASFLTAAARRAGVIPRPWS
GGVEALPGTCSAVSFTGSYTSATATNFPASQTPVTGTGTATGTSPPTTSTTAQPPSTTT
ACAIAPQVTVNFVARVVTNYGDTVKLVGNVDKLGNWNPGSGVVFSASDYQANNPV
WKGSVVLSAGQSIQYKYVKVLSDGTVKWEADPNRTYSVPRSCATAVTRSDTWQT
SEQ ID NO: 20; GA-4688 (PoxGA5) from *Penicillium oxalicum*

APQLSPRATASLDAWLATETTFSLNGILNNIGASGAYAKSAKNGVVIASPSTSSPNYY
YTWSRDSALTLKVLIDLFRNGNLDLQTVIEEYINAQATLQTVSNPSGDLSSGAGLGEP
KFNVDLSAFTDGWGRPQRDGPALRAISLIEFGNWLIDNGYSSYAINNVWPIVRNDLS
YVAQYWSQTGFDLWEEVNSMSFFTVASQHRSLVEGSAFAKRVGASCSWCDSQAPQI
LCYMQTFWTGSYMNANTGGGRSGKDANTVLASIHTFDPEATCDDITFQPCSPRALA
NHKVYTDSFRSVYSINSGIAQGVAVAVGRYPEDSYYNGNPWFLSNLAAAEQLYDAI
YQWNKIGSITITSTSLAFFKDIYSSAAVGTYASGSSTFTAIISAVKTYADGYVSIVQAH
SYTNGSLSEQYDKSTGLSLSARDLTWSYAALLTANMRRNGVVPPSWGASSANTVPS
SCSMGSAAGTYATPTATSWPSTLTSGTPGSTTSTPATSTTSTTSTSACTTPTSVAVTFD
EIATTTYGENVYIVGSISQLGSWNTANAIALSASQYTSSKHLWYVTINLPAGTTFQYK
YIRKESDGSIVWESDPNRSYTVPATCGTTTATENDTWR

The AA that performed better in combination with TrGA, or better in a blend with a different GA, are listed in the following table:

| **Name** | **Abbr.** | **Source organism** | **SEQ ID NO*** |
|---|---|---|---|
| AA-1704 | AcAA | *Aspergillus clavatus* | 21 |
| AA-1708 | AtAA | *Aspergillus terreus* | 22 |
| AA-2115 | AfuAmy1 | *Aspergillus fumigatus* Af293 | 23 |
| AA-2205 | NfiAmy1 | *Neosartorya fischeri* | 24 |
| AA-2285 | TemAmy1 | *Talaromyces emersonii* | 25 |
| AA-2301 | PfuAmy1 | *Penicillium funiculosum* | 26 |
| AA-2303 | PfuAmy3 | *Penicillium funiculosum* | 27 |
| AA-2506 | ApuAmy1 | *Aureobasidium pullulans* | 28 |
| AA-2522 | LstAmy1 | *Lipomyces starkeyi* | 29 |
| AA-2676 | OsaAmy2 | *Oryza sativa* Japonica Group | 30 |
| AA-2940 | AacAmy2 | *Aspergillus aculeatus* | 31 |
| AA-3238 | TleAmy1 | *Talaromyces leycettanus* | 32 |
| AA-3239 | TauAmy1 | *Thermoascus aurantiacus* | 33 |
| AA-3937 | BhaAmy3 | *Brevibacterium halotolerans* strain XFB-BI | 34 |

The amino acid sequences are shown, below:
SEQ ID NO: 21; AA-1704 (AcAA) from *Aspergillus clavatus* 
LTPAEWRGQSIYFLITDRFARTDGSTTAPCDLSQRAYCGGSWQGIIKQLDYIQGMGFT
AIWITPITEQIPQDTAEGSAFHGYWQKDIYNVNSHFGTADDIRALSKALHDRGMYLM
IDVVANHMGYNGPGASTDFSTFTPFNSASYFHSYCPINNYNDQSQVENCWLGDNTV
ALADLYTQHSDVRNIWYSWIKEIVGNYSADGLRIDTVKHVEKDFWTGYTQAAGVY
TVGEVLDGDPAYTCPYQGYVDGVLNYPIYYPLLRAFESSSGSMGDLYNMINSVASD
CKDPTVLGSFIENHDNPRFASYTKDMSQAKAVISYVILSDGIPIIYSGQEQHYSGGNDP
YNREAIWLSGYSTTSELYKFIATTNKIRQLAISKDSSYLTSRNNPFYTDSNTIAMRKGS
GGSQVITVLSNSGSNGGSYTLNLGNSGYSSGANLVEVYTCSSVTVGSDGKIPVPMAS
GLPRVLVPASWMSGSGLCGSSSTTTLVTATTTPTGSSSSTTLATAVTTPTGSCKTATT
VPVVLEESVRTSYGENIFISGSIPQLGSWNPDKAVALSSSQYTSSNPLWAVTLDLPVG
TSFEYKFLKKEQNGGVAWENDPNRSYTVPEACAGTSQKVDSSWR
SEQ ID NO: 22; AA-1708 (AtAA) from *Aspergillus terreus* 
LTPAEWRSQSIYFLLTDRFGRTDNSTTAACDTSDRVYCGGSWQGIINQLDYIQGMGF
TAIWITPVTGQFYENTGDGTSYHGYWQQDIYDLNYNYGTAQDLKNLANALHERGM
YLMVDVVANHMGYDGAGNTVDYSVFNPFSSSSYFHPYCLISNYDNQTNVEDCWLG
DTTVSLPDLDTTSTAVRNIWYDWVADLVANYSIDGLRVDTVKHVEKDFWPGYNSA
AGVYCVGEVYSGDPAYTCPYQNYMDGVLNYPIYYQLLYAFESSSGSISDLYNMISSV
ASSCKDPTLLGNFIENHDNPRFASYTSDYSQAKNVITFIFLSDGIPIVYAGQEQHYSGG
SDPANREATWLSGYSTSATLYTWIATTNQIRSLAISKDAGYVQAKNNPFYSDSNTIA
MRKGTTAGAQVITVLSNKGASGSSYTLSLSGTGYSAGATLVETYTCTTVTVDSSGNL
PVPMTSGLPRVFVPSSWVNGSALCNTECTAATSISVLFEELVTTTYGENIYLSGSISQL
GSWNTASAVALSASQYTSSNPEWYVSVTLPVGTSFQYKFIKKGSDGSVVWESDPNR
SYTVPAGCEGATVTVADTWR
SEQ ID NO: 23; AA-2115 (AfuAmy1) from *Aspergillus fumigatus* Af293 
LTPAEWRSQSIYFLLTDRFGREDNSTTAACDVTQRLYCGGSWQGIINHLDYIQGMGF
TAIWITPVTEQFYENTGDGTSYHGYWQQNIHEVNANYGTAQDLRDLANALHARGM
YLMVDVVANHMGYNGAGNSVNYGVFTPFDSATYFHPYCLITDYNNQTAVEDCWL
GDTTVSLPDLDTTSTAVRSIWYDWVKGLVANYSIDGLRIDTVKHVEKDFWPGYNDA
AGVYCVGEVFSGDPQYTCPYQNYLDGVLNYPIYYQLLYAFQSTSGSISNLYNMISSV
ASDCADPTLLGNFIENHDNPRFASYTSDYSQAKNVISFMFFSDGIPIVYAGQEQHYSG
GADPANREAVWLSGYSTSATLYSWIASTNKIRKLAISKDSAYITSKNNPFYYDSNTLA
MRKGSVAGSQVITVLSNKGSSGSSYTLSLSGTGYSAGATLVEMYTCTTLTVDSSGNL
AVPMVSGLPRVFVPSSWVSGSGLCGDSISTTATAPSATTSATATRTACAAATAIPILFE
ELVTTTYGESIYLTGSISQLGNWDTSSAIALSASKYTSSNPEWYVTVTLPVGTSFEYKF
VKKGSDGSIAWESDPNRSYTVPTGCAGTTVTVSDTWR
SEQ ID NO: 24; AA-2205 (NfiAmy1) from *Neosartorya fischeri* 
LTPAEWRSQSIYFLLTDRFGREDNSTTAACDVTQRLYCGGSWQGIINHLDYIQGMGF
TAIWITPVTQQFYENTGDGTSYHGYWQQNIYEVNSNYGTAQDLRKLADALHARGM
YLMVDVVANHMGYDGAGNSVDYSVFTPFDSSTYFHTYCLISDYNNQNNVEDCWLG
DTTVSLPDLDTTNTAVRTIWYDWVKGLVANYSIDGLRIDTVKHVEKDFWPDYNDA
AGVYCVGEVFSGDPSYTCPYQNYMDGVLNYPIYYQLLYAFQSTSGSISNLYNMISSV
DSDCADPTLLGNFIENHDNPRFASYTSDYSQAKNVISFMFFSDGIPIVYAGQEQHYSG
GADPANREAVWLSGYSTSATLYSWIASTNKIRKLAISKDSAYITSKNNPFYYDSNTLA
MRKGSVAGSQVITVLSNKGSSGSSYTLSLSGTGYSAGATLVEMYTCTTLTVDSSGNL
AVPMASGLPRVLVPSSWVSGSGLCGDSISTIATTTTSTTKTTTVATTTACASATALPIL
FEELVTTTYGETIYLTGSISQLGNWDTSSAIALSASKYTSSNPEWYATVTLPVGTSFQY
KFFKKESDGSIVWESDPNRSYTVPAGCAGTTVTVSDTWR
SEQ ID NO: 25; AA-2285 (TemAmy1) from *Talaromyces emersonii* 
LTPAEWRKQSIYFLLTDRFGRADNSTTAACDVTERIYCGGSWQGIINHLDYIQGMGF
TAIWISPVTEQLPQNTGEGEAYHGYWQQEIYTVNSNFGTSDDLLALSKALHDRGMY
LMVDVVANHMGYDGDGDSVDYSVFNPFNSSSYFHPYCLITDYSNQTDVEDCWLGD
TTVSLPDLNTTETVVRTIWYDWVADLVSNYSIDGLRIDTVKHVEKSFWPGYNSAAG
VYCVGEVLDGDPSYTCPYQDYLDGVLNYPIYYQLLYAFESSSGSISNLYNMINSVAS
ECSDPTLLGNFIENHDNPRFASYTSDYSLAKNVIAFIFFSDGIPIVYAGQEQHYNGGND
PYNREATWLSGYSTTAELYTFIATTNAIRSLAISVDSEYLTYKNDPFYYDSNTLAMRK
GSDGLQVITVLSNLGADGSSYTLTLSGSGYSSGTELVEAYTCTTVTVDSNGDIPVPME
SGLPRVFLPASSFSGSSLCSSSPSPTTTTSTSTSTTSTACTTATAVAVLFEELVTTTYGE
NVYLSGSISQLGDWNTDDAVALSAANYTSSNPLWYVTVTLPVGTSFEYKFIKKEENG
DVEWESDPNRSYTVPTACTGATETIVDTWR
SEQ ID NO: 26; AA-2301 (PfuAmy1) from *Penicillium funiculosum* 
LSAAEWRSQSIYFLLTDRFARTDGSTSAACDLSQRIQAYCGGSWQGIIDHLDYIQGM
GFTAVWITPITKQMPQTTSEGTGFHGYWQQDIYSVNPNFGTADDIKALSKAIHDRGM
YLMIDVVANHMGYNGAGSSTDFSVFNPFNSASYFHSYCSISDYNNQNQVENCWLGD
DTVSLTDLNTQSDQVRTIWYSWVKDLVANYTVDGLRIDTVKHVEKDFWTGYSQAA
GVYTLGEVLHGDPAYTCPYQGYVDGVFNYPIYYPLLNAFKSSSGSISSLVSMINSVSS
DCKDPTLLGSFIENHDNPRFPSYTSDMSQAKSVIGYVFFADGIPTIYSGQEQHYAGAN
 
DPYNREAIWLSGYATDSELYKFIATANEIRKLAISKDSSYLTTRNNAFYTDSNTIAMR
KGTSGSQVITVLSNSGSSGGSYTLNLNNHGYSSGAQLVELYTCASVQVDSSGNMPVP
MASGLPRVLVPGSWATGSGLCGTSSGTPSKTTTLITTTSQVSSSTSSTCVAATSLPIAF
TEKVTTSYGESVFITGSISQLGNWNAANAVALSASQYTSANPVWTVSLDLPVGTTFQ
YKYIKKEQDGSVVWESDPNRSYTVSSGCTGVKQAVSDSWR
SEQ ID NO: 27; AA-2303 (PfuAmy3) from *Penicillium funiculosum* 
LTADEWRSQSIYFLLTDRFGLTSNSTTASCDVADGLYCGGSWQGVINHLDYIQGMGF
TAIWITPVTENFEGDTSDGEAYHGYWQQNAYATNSHYGASSDLLKLSEALHARGMY
LMVDIVVNNMAYDGAGTSVDYSIFNPFPSESYYHSYCLINYNTYNATDWDDCWEG
DTIVSLPDLDTTQTYVKDTWNTWVKSFVANYSIDGLRIDSALHIQQDFFTAFEEAAG
VYCIGELDYGDPAVVCPYQSVLSGVLNYPIYWQLLYAFESSSGSISNLYNMINTVKS
DCADTSLLGNFIENHDNPRFAYYTSDYSEAKNVISFIFLTDGIPILYYGQEQHYSGGNI
PLNREPLWTSDYSTDAQLYTYTKTSNAIRSLAIAKDSAYLTYQNYPIYQDSNTIAMRK
GTTGLQLVTVLSNLGANGSSYTLTLSGSGYTSGTVVTELYTCTNVTVSSSGTIAVPM
ASGSPRAFLPWSSVSGSSLCNSVSSGCTAASTVAVTFEEVVTTTYGQEVYLTGSISQL
GSWSTSSAVLLSAAQYTSSDPVWTVTVNLPAGESFEYKFIIVNSDGTVTWESDPNRS
YTVPTGCQGLTATVDDTWR
SEQ ID NO: 28; AA-2506 (ApuAmy1) from *Aureobasidium pullulans* 
LTPAQWRSQSIYQVLTDRFARTDGSTTASCDVNKYCGGSFQGIIKKLDYIQQMGFTAI
WISPVVKNIYSSGQDGDSYHGYWAQDIYQVNTNFGSAADLVSLSKALHDRGMYLM
VDIVTNHMGYNGCGNCVDYSIYNPFNSQSYYHPFCLINYNDQTSVEQCWAGDNTVS
LPDLRTEDSNVLSMWNTWIKQLVFNYTIDGLRIDSAKSVDKAFYQPFQQAASVYAV
GEVYDGDPNYFCDYQNYLDGMLNYPTYYWITQAFQSTSGSISNLYNGINTMKSTCK
DTTLLGSFMENHDVARFASLTSDYALAKNAIAFTMLADGIPIIYQGQEQHFSGSSVPN
NREALWLSGYPTSSQLYPFIATVNKIRKQAIKQDTGYLTYKAYPVYSDASTIVMRKG
TTGSQVIGVFTNKGSSGSSSFTLSSSASGFTAGQAVTDVLSCTSYTADSNGNIAININA
GAPRVLYPTSKLTGSGLCSGSSSTSGTPTTIKTSAVSGGCSTPTAVAVTFTDKVTTQY
GQTIKLAGSIPQLGSWNAANAVTLSSAGYTASNPVWSGTVNIPAGQAFSYKFIKVNS
DGSVTWESDPNHSYTVPASCGVTTASVSNTWQG
SEQ ID NO: 29; AA-2522 (LstAmy1) from *Lipomyces starkeyi* 
YILRRDCTTVTVLSSPESVTSSNHVQLASHEMCDSTLSASLYIYNDDYDKIVTLYYLT
SSGTTGSVTASYSSSLSNNWELWSLSAPAADAVEITGASYVDSDASATYATSFDIPLT
TTTTSSSSASATSTSSLTTTSSVSISVSVPTGTAANWRGRAIYQIVTDRFARTDGSTTY
LCDVTDRVYCGGSYQGIINMLDYIQGMGFTAIWISPIVENIPDDTGYGYAYHGYWM
KDIFALNTNFGTADDLIALATELHNRGMYLMVDIVVNHFAFSGSHADVDYSEYFPYS
SQDYFHSFCWITDYSNQTNVEQCWLGDDTVPLVDVNTQLDTVKSEYQSWVQELIAN
YSIDGLRIDTVKHVQMDFWAPFQEAAGIYAVGEVFDGDPSYTCPYQENLDGVLNYP
VYYPVVSAFESVSGSVSSLVDMIDTLKSECTDTTLLGSFLENQDNPRFPSYTSDESLIK
NAIAFTMLSDGIPIIYYGQEQGLNGGNDPYNREALWLTGYSTTSTFYKYIASLNQIRN
QAIYKDDTYLTYQNWVIYSDSTTIAMRKGFTGNQIITVLSNLGTSGSSYTLTLSNTGY
TASSVVYEILTCTAVTVDSSGNLAVPMSSGLPKVFYQESQLVGSGICSM
SEQ ID NO: 30; AA-2676 (OsaAmy2) from *Oryza sativa* Japonica Group 
DKILFQGFNWESWRQSGGWYNLLMGKVDDIVAAGVTHVWLPPPSHSVSTQGYMPG
RLYDLDASRYGTSMELKSLISALHGKGIQAIADVVINHRCADYKDSRGIYCIFEGGTP
DGRLDWGPHMICRDDTQFSDGTGNLDTGADFAAAPDIDHLNGVVQRELTDWLLWL
KSDEVGFDAWRLDFARGYSPEVAKVYIEGTTPVGLAVAELWDSMAYGGDGKPEYN
QDAHRQALVDWVDRVGGTASAGMVFDFTTKGIMNTAVEGELWRLIDQQGKAPGVI
GWWPAKAVTFVDNHDTGSTQQMWPFPSDKVMQGYAYILTHPGNPCIFYDHFFDWG
LKEQIAALVAVRQRNGVTATSSLKIMLHDADAYVAEIDGKVVMKIGSRYDVSSLIPP
GFHLAAHGNGYAVWEKSAAAAADHRTSSSASL
SEQ ID NO: 31; AA-2940 (AacAmy2) from *Aspergillus aculeatus* 
AEWRTQSIYFLLTDRFGRTDNSTTATCNTGDQVYCGGTWQGIINHLDYIQGMGFTA
VWISPVTEQLSANTADGESYHGYWQQKIYSLNSNFGTADDLKALSAALHERDMYL
MVDVVPNHMGYAGSGDSVDYSVFDAFDSSSYFHSYCLITDWDDIDQVRTCWEGDTI
VSLPDLYTTQSDVRTIWYDWIEQLVANYSIDGLRIDSALEVEPDFFTGYVSAAGVYS
VGEIFNGDPATACPYQGYLDGVLNYPIYFQLLYAFESSSGSISDLYNMINSVASDCSD
PTLLGNFIENHDNARFAYYTSDYSQAKNVLSFLFLSDGIPIVYAGEEQHYSGSGVPYN
REATWLSGYSTTAELYQWIATTNAIRKLAISLDSNYITYKNNPFYTDSNTIAMRKGSD
NLQVITILSNRGSSSSSYTLTLTGTGYAAGTTLIEAYTCTTLTVSSSGSIAVPMASGLPR
VYLPASSVNKGSLCGGGTSATTATTTTTLKTTTTTTSTKTTTTSCTATTTSLPITFIELV
TTTYGEEIYLTGSIAALGNWATTASGRIALSAANYSASYPEWSATVSVPVGTSFEYKF
FKVGTDGSTITWESDPNRVYTVTATACAGATATVVDSWR
SEQ ID NO: 32; AA-3238 (TleAmy1) *Talaromyces leycettanus* 
LAPAEWRKQSIYFLLTDRFGRTDNSTTATCNVSDRVYCGGSWQGIINHLDYIQGMGF
TAVWISPVTEQLPQDTGDGAAYHGYWQQRIYELNANFGTESDLKALATALHDRGM
YLMLDVVANHMGYAGAGNTVDYSVFDPFDSSSYFHPYCLISDYSNQTNVEDCWLG
DTTVSLPDLNTTETAVQNIWYNWVAGLVANYSVDGLRIDTVKHVQKPFWPGYNKA
AGVYCVGEVLNGDPSYTCDYQNYLDAVLNYPIYFQLLYAFESSSGSIANLYNMINSV
ASVCVDPTLLGNFIENHDNPRFAYYTSDYSQAKNVIAYIFLADGIPIVYAGQEQHYSG
GNDPYNREATWLSGYSTSAELYTFIATTNQIRKLAISRDSNYLTSRNNPFYYDSNTLA
MRKGSSGSQVITVLSNLGSSGSSYTLTLSNTGYSSGTSLTELHTCTSVTVDSSGNIAVP
MASGSPRVLVPSSWINGSGLCSGSGTTGCTAATSVPVLFEETVTTTYGENIFISGSISQ
LGDWDTSQAVALSASQYTASDPLWEVTIDLPVGTSFEYKFIKVEPSGTVVWESDPNR
QYTVPTACTGTTETVVATWR
SEQ ID NO: 33; AA-3239 (TauAmy1) from *Thermoascus aurantiacus* 
ATPAQWRSRSVYFLLTDRFARSDGSTTAACDTSARLDYIQGMGFTAIWISPVTEQLP
QDTGDGTAYHGYWQQDIYSLNPNFGTADDLRALADALHARGMYLMVDVVANHM
GYAGPGNSVDYSVFNPFNKQEYFHPYCEITNYDDQSNVEDCWLGDTIVSLPDLNTTR
SDVEDIWYSWVRALVSNYSVDGLRIDTVKHVQKDFWPGYNDAAGVYCVGEVFDG
DPSYTCDYQNYLDGVLNYPMYYPLLRAFSSTSGSISDLYNMINTVKAQCADSTLLGT
FVENHDVPRFASYTSDIALAKNAIAFTILSDGIPIIYAGQEQHYSGGNDPANREAVWL
SGYSTTSELYQFIAVSNQIRNYAIYVDEGYLTYKAWPIYQDSHTLAIRKGFDGNQVIT
VLSNLGSSGSSYTLSLSGTGYAAGQQVTEIYSCTDVTADSNGNIAVSMGGGLPKAFF
PTAKLAGSGICWK
SEQ ID NO: 34; AA-3937 (BhaAmy3) from *Brevibacterium halotolerans* strain XFB-BI

GPAAANAETQNTSNELTAPSIKSGTILHAWNWSFNTLKHNMKDIHDAGYTAIQTSPI
NQVKEGNQGNKSMSNWYWLYQPTSYQIGNRYLGTEQEFKEMCAAAEEYGVKVIV
DAVINHTTSDYAAISNEIKSIPNWTHGNTQIKNWSDRWDVTQNSLLGLYDWNTQNT
QVQSYLKRFLERALNDGADGFRYDAAKHIELPDDGNYGSQFWPNITNTSAEFQYGEI
LQDSASRDAAYANYMNVTASNYGHSIRSALKNRNLSVSNISHYASEVSADKLVTWV
ESHDTYANDEEESTWMSDDDIRLGWAVIASRSGSTPLFFSRPEGGGNGVRFPGKSQI
GDRGSALFEDQAITAVNRFHNVMDGQPEELSNPNGNNQIFMNQRGSHGVVLANAGS
SSVTINTSTKLPDGRYDNKAGNGSFQVTDGKLTGTINARSVAVLYSDDIANAPHVFL
ENVKTGVTHSFNDQLTITLRADANTTKAVYQINNGQETVFKDGDQLTIGKGDPFGTT
YTITLTGTNSDGVTRTQEYSFVKREPSAAKTIGYQNPNHWGQVNAYIYKHDGGRAL
ELTGSWPGKAMIKNADGIYTLTLPADTDTTNAKVIFNNGSAQVPGQNQPGFDYVQN
GLYNDSGLSGSLPH

GA/AA combinations that performed better than TrGA/AkAA are listed in the following table:

| **AA** | **GA** |
|---|---|
| AA-1708 | GA-3317 |
| | GA-3298 |
| | GA-2040 |
| | GA-3280 |
| | GA-2441 |
| | GA-1805 |
| | GA-2439 |
| | GA-4686 |
| | GA-3301 |
| | GA-2331 |
| | GA-3275 |
| AA-3238 | GA-3317 |
| | GA-3280 |
| | GA-3298 |
| | GA-4688 |
| | GA-2441 |
| | GA-4686 |
| | GA-2040 |
| AA-2285 | GA-3317 |
| | GA-2441 |
| | GA-3298 |
| | GA-3280 |
| AA-2522 | GA-3317 |
| | GA-3298 |
| | GA-2439 |
| AA-3239 | GA-3298 |
| | GA-1805 |
| | GA-3317 |
| | GA-2439 |
| AA-2303 | GA-3298 |
| | GA-3317 |
| | GA-2439 |
| | GA-3301 |
| | GA-2441 |
| | GA-3280 |
| AA-2940 | GA-3317 |
| AA-1704 | GA-3298 |

In some embodiments, the compositions and methods include a granular starch-converting α-amylase having an amino acid sequence with at least 95%, at least 96%, at least 97%, at least 98%, or even at least 99% amino acid sequence identity to SEQ ID NO: 22, and a granular starch-converting glucoamylase having at least at least 95%, at least 96%, at least 97%, at least 98%, or even at least 99% amino acid sequence identity to any one of SEQ ID NOs: 18, 16, 4, 13, 8, 3, 7, 19, 17, 5 or 12,

In some embodiments, the compositions and methods include a granular starch-converting α-amylase having an amino acid sequence with at least at least 95%, at least 96%, at least 97%, at least 98%, or even at least 99% amino acid sequence identity to SEQ ID NO: 32, and a granular starch-converting glucoamylase having at least 95%, at least 96%, at least 97%, at least 98%, or even at least 99% amino acid sequence identity to any one of SEQ ID NOs: 18, 13, 16, 20, 8, 19, or 4,

In some examples, the compositions and methods include a granular starch-converting α-amylase having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98%, or even at least 99% amino acid sequence identity to SEQ ID NO: 25, or to an active fragment, thereof, and a granular starch-converting glucoamylase having at least 85%, at least 86%, at least 87%, at least 88%, at least 89%, at least 90%, at least 91%, at least 92%, at least 93%, at least 94%, at least 95%, at least 96%, at least 97%, at least 98%, or even at least 99% amino acid sequence identity to any one of SEQ ID NOs: 18, 8, 16, or 13, or an active fragments, thereof.

In some examples, the compositions and methods include a granular starch-converting α-amylase having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98%, or even at least 99% amino acid sequence identity to SEQ ID NO: 29, or to an active fragment, thereof, and a granular starch-converting glucoamylase having at least 85%, at least 86%, at least 87%, at least 88%, at least 89%, at least 90%, at least 91%, at least 92%, at least 93%, at least 94%, at least 95%, at least 96%, at least 97%, at least 98%, or even at least 99% amino acid sequence identity to any one of SEQ ID NOs: 18, 16, or 7, or an active fragments, thereof.

In some examples, the compositions and methods include a granular starch-converting α-amylase having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98%, or even at least 99% amino acid sequence identity to SEQ ID NO: 33, or to an active fragment, thereof, and a granular starch-converting glucoamylase having at least 85%, at least 86%, at least 87%, at least 88%, at least 89%, at least 90%, at least 91%, at least 92%, at least 93%, at least 94%, at least 95%, at least 96%, at least 97%, at least 98%, or even at least 99% amino acid sequence identity to any one of SEQ ID NOs: 16, 3, 18, or 7, or an active fragments, thereof.

In some embodiments, the compositions and methods include a granular starch-converting α-amylase having an amino acid sequence with at least 95%, at least 96%, at least 97%, at least 98%, or even at least 99% amino acid sequence identity to SEQ ID NO: 27, and a granular starch-converting glucoamylase having at least 95%, at least 96%, at least 97%, at least 98%, or even at least 99% amino acid sequence identity to any one of SEQ ID NOs: 16, 18, 7, 17, 8, or 13,

In some examples, the compositions and methods include a granular starch-converting α-amylase having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98%, or even at least 99% amino acid sequence identity to SEQ ID NO: 31, or to an active fragment, thereof, and a granular starch-converting glucoamylase having at least 85%, at least 86%, at least 87%, at least 88%, at least 89%, at least 90%, at least 91%, at least 92%, at least 93%, at least 94%, at least 95%, at least 96%, at least 97%, at least 98%, or even at least 99% amino acid sequence identity to SEQ ID NO: 18, or an active fragments, thereof.

In some examples, the compositions and methods include a granular starch-converting α-amylase having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98%, or even at least 99% amino acid sequence identity to SEQ ID NO: 21, or to an active fragment, thereof, and a granular starch-converting glucoamylase having at least 85%, at least 86%, at least 87%, at least 88%, at least 89%, at least 90%, at least 91%, at least 92%, at least 93%, at least 94%, at least 95%, at least 96%, at least 97%, at least 98%, or even at least 99% amino acid sequence identity to SEQ ID NO: 16, or an active fragments, thereof.

In some embodiments, the compositions and methods include a plurality of the granular starch-converting glucoamylase and/or α-amylase enzymes described, herein.

In some embodiments, the compositions and methods further include other enzymes, such as other α-amylases and glucoamylases, including other granular starch hydrolyzing enzymes. In some embodiments, the addition enzyme is selected from a cellulase, a glucanase, a xylanase, a phytase, a protease, a trehalase, and a pullulanase.

In some embodiments, the granular starch has a DS of between 5 - 60%; 10 - 50%; 15 - 45%; 15- 30%; 20 - 45%; 20 - 30% and also 25 - 40%. The contacting step with glucoamylase and/or α-amylase is conducted at a pH range of 3.0 to 7.0; 3.0 to 6.5; 3 to 5.5; 3.5 t0 4.5; 3.5 to 7.0; 3.5 to 6.5; 4.0 to 6.0 or 4.5 to 5.5. The slurry is held in contact at a temperature at or below the starch gelatinization temperature of the granular starch. In some embodiments, this temperature is held between 45°C and 70°C; in other embodiments, the temperature is held between 50°C and 70°C; between 55°C and 70°C; between 60°C and 70°C, between 60°C and 65°C; between 55°C and 65°C and between 55°C and 68°C. In further embodiments, the temperature is at least 45°C, 48°C, 50°C, 53°C, 55°C, 58°C, 60°C, 63°C, 65°C and 68°C. In other embodiments, the temperature is not greater than 65°C, 68°C, 70°C, 73°C, 75°C and 80°C.

The initial starch gelatinization temperature ranges for a number of granular starches which may be used in accordance with the processes herein can include, but are not limited to barley (52°C to 59°C), wheat (58°C to 64°C), rye (57°C to 70°C), corn (62°C to 72°C), high amylose corn (67°C to 80°C), rice (68°C to 77°C), sorghum (68°C to 77°C), potato (58°C to 68°C), tapioca/cassava (59°C to 69°C) and sweet potato (58°C to 72°C). (J.J.M. Swinkels pg 32 - 38 in Starch Conversion Technology, Eds Van Beynum et al., (1985) Marcel Dekker Inc. New York and The Alcohol Textbook 3rd ED. A Reference for the Beverage, Fuel and Industrial Alcohol Industries, Eds Jacques et al., (1999) Nottingham University Press, UK).

In the contacting step, the slurry may be held in contact with the present enzymes for a period of 5 minutes to 48 hours; and also for a period of 5 minutes to 24 hours. In some embodiments the period of time is between 15 minutes and 12 hours, 15 minutes and 6 hours, 15 minutes and 4 hours and also 30 minutes and 2 hours. Total ethanol fermentation time typically requires 30-70 hours, for example, 40-70, 30-60, 50-70, 30-50, or similar hours.

During the contacting step between 25 - 90% or more of the granular starch is solubilized to produce saccharides comprising dextrin, oligosaccharides, and smaller sugars like glucose. In some embodiments, greater than 20%, 25%, 30%, 35%, 40%, 45%, 50%, 55%, 60%, 65%, 70%, 75%, 80%, 85% and 90% of the granular starch is solubilized.

After contacting the granular starch with the α-amylase and glucoamylase for a period of time as indicated above, a soluble starch substrate (mash) is obtained which comprises greater than 10%, 15%, 20%, 25%, 30%, 35%, 40%, 45%, 50%, 55%, 60%, 65%, 70%, 75%, 80%, 90%, 95% and 97% glucose.

After the contacting step which results in the production of a mash comprising glucose, the mash is typically subjected to fermentation with a fermenting microorganism (e.g. an ethanol-producing microorganism). The fermentation can be done simultaneously with the contacting step during which the produced glucose can be converted immediately to the end product by the fermenting microorganism. In this case, the amount of glucose that accumulates in the mash will be much lower, as it is rapidly converted to an end of fermentation product.

In some embodiments the fermenting organism is yeast, optionally recombinant yeast. Examples of yeast include but are not limited to a Saccharomyces sp., a Candida sp., a Pichia sp., a Dekkera sp., an Hanseniaspora sp., a Pseudozyma sp., a Sacharromycodes sp., a Zygosaccharomyces sp., a Zygoascus sp., an Issatchenkia sp., a Williopsis sp., and a Brettanomyces sp. Particular yeast include but are not limited to Saccharomyces cerevisiae, Torulaspora delbrueckii, Brettanomyces bruxellensis, Zygosaccharomyces bailii, Debaryomyces hansenii, and Zygosaccharomyces rouxii.

In some embodiments the fermenting organism is filamentous fungi, optionally recombinant filamentous fungi. Examples of filamentous fungi include but are not limited to a Trichoderma sp., an Aspergillus sp., a Penicillium sp., and a Myceliopthora sp. (such as C1 from Dyadic).

In some embodiments the fermenting organism is a bacterium, optionally a recombinant bacterium. Preferred bacterial fermenting organisms include an Escherichia sp., a Zymomonas sp., a Bacillus sp., a Corynebacterium sp., a Brevibacterium sp., a Streptomyces sp., and a Klebsialla sp.. In some embodiments, the bacterium is capable of producing an alcohol, e.g., ethanol, butanol, methanol, propanol etc.

Improved strains of ethanologenic microorganisms, which can withstand higher temperatures, for example, are known in the art and can be used. See Liu et al. (2011) Sheng Wu Gong Cheng Xue Bao 27(7): 1049-56. Commercial sources of yeast include ETHANOL RED^{®} (LeSaffre); THERMOSACC^{®} (Lallemand); RED STAR^{®} (Red Star); FERMIOL^{®} (DSM Specialties); and SUPERSTART^{®} (Alltech).

In some embodiments the fermenting organism expresses enzymes such as the granular starch-converting glucoamylases and/or converting α-amylases described, herein, other glucoamylases and/or α-amylases or starch degrading enzymes, such as pullanase and/or trehalase. Other enzymes include phytase, cellulase, xylanase, glucanase, xylose reductase, xylitol dehydrogenase, protease, and the like.

Use of the present granular starch-converting glucoamylases and α-amylases is not restricted to production of a particular end of fermentation (EOF) product. In some embodiments, the EOF may be, but is not limited to, metabolites, such as citric acid, lactic acid, succinic acid, acetic acid, monosodium glutamate, gluconic acid, sodium gluconate, calcium gluconate, potassium gluconate, itaconic acid and other carboxylic acids, glucono delta-lactone, sodium erythorbate, glutamic acid, tryptophan, threonine, methionine, lysine and other amino acids, omega-3 fatty acid, isoprene, 1,3-propanediol, ethanol, methanol, propanol, butanol, other alcohols, and other biochemicals and biomaterials.

Prior to subjecting the mash to fermentation, the mash may be further exposed to an aqueous solution comprising, for example, backset and/or corn steep, and adjusted to a pH in the range of pH 3.0 to 6.0; pH 3.5 to 5.5, or pH 4.0 to 5.5. In this embodiment, the % DS of the mash may be diluted. For example, the DS of the diluted mash maybe between 5 to 35%; 5 to 30%; 5 to 25%; 5 to 20%; 5 to 20%; 5 to 15%; and 5 to 10% less than the %DS of the slurry in the contacting step. In one non-limiting example, if the %DS of the slurry in the contacting step is approximately 32% and the mash is further exposed to a diluting aqueous solution which dilutes the DS between 5 to 10%, the DS of the mash to be fermented will be between 22% and 27%. In some specific embodiments, if the DS of the contacting slurry is between 30 to 35 %, the DS of the diluted slurry will be about 20 to 30%.

In a specific embodiment, mash comprising at least 10% glucose is then subjected to fermentation processes using fermenting microorganisms as described above. These fermentation processes are described in The Alcohol Textbook 3rd ED, A Reference for the Beverage, Fuel and Industrial Alcohol Industries, Eds Jacques et al., (1999) Nottingham University Press, UK.

In some embodiments, contacting the granular starch with the α-amylase and glucoamylase is performed simultaneously with fermentation by the fermenting microorganism. During this process the glucose content (or that of other fermentable sugars) remains low because it is simultaneously converted to end product by the fermenting microorganisms as described above.

As noted, one EOF product that can be produced using the present compositions and methods is an alcohol product, such as ethanol. The end product produced according to the process may be separated and/or purified from the fermentation media. Methods for separation and purification are known, for example by subjecting the media to extraction, distillation and column chromatography.

In further embodiments, the mash may be separated at any time in fermentation, but preferably at the end of fermentation, and even more preferably after removal of end product ethanol by distillation, by for example centrifugation into the liquid phase and solids phase The alcohol may be recovered by means such as distillation and can be further purified by molecular sieve dehydration or ultra-filtration.

In some embodiments, the yield of ethanol will be greater than 8%, 10%, 12%, 14%, 16% and 18% by volume. The ethanol obtained may be used as a fuel ethanol, potable ethanol or industrial ethanol.

In addition to the EOF product, the present granular starch-converting glucoamylases and α-amylases may offer advantages in the production or quality of fermentation co-products such as distillers dried grains (DDG) and distiller's dried grain plus solubles (DDGS), which may be used as an animal feed or other applications.

### EXAMPLES

### Example 1

### Evaluation of GA/AA blends in Simultaneous Saccharification and Fermentation

A number of glucoamylases (GA) and α-amylases (AA) were tested in combination as enzyme blends for use in simultaneous saccharification and fermentation using a corn flour substrate.

The GA used are listed in the following table:

| **Name** | **Abbr.** | **Source organism** | **SEQ ID NO** |
|---|---|---|---|
| GA-1805 | AteGA1 | *Aspergillus terreus* | 3 |
| GA-2040 | AfuHT3 | *Aspergillus fumigatus* | 4 |
| GA-2331 | NfiGA1 | *Neosartorya fischeri* | 5 |
| GA-2437 | AfuGA2 | *Neosartorya fumigata* | 6 |
| GA-2439 | PmaGA1 | *Penicillium marneffei* | 7 |
| GA-2441 | TstGA2 | *Talaromyces stipitatus* | 8 |
| GA-2442 | MacGA1 | *Metarhizium acridum* | 9 |
| GA-2578 | ScoGA1 | *Schizophyllum commune* | 10 |
| GA-2722 | Tat GA2 | *Trichoderma atroviridis; Hypocrea atroviridis* | 11 |
| GA-3275 | BadGA1 | *Bjerkandera adusta* | 12 |
| GA-3280 | GspGA1 | *Ganoderma spp* | 13 |
| GA-3283 | TveGA3 | *Termetes versicolor* | 14 |
| GA-3294 | HsuGA3 | *Hypholoma sublateritium* | 15 |
| GA-3298 | FmeGA1 | *Fomitiporia mediterranea* | 16 |
| GA-3301 | PstGA2 | *Punctularia strigosozonata* | 17 |
| GA-3317 | PbrGA1 | *Phlebia brevispora Nakasone* | 18 |
| GA-4686 | SzeGA2 | *Sarocladium zeae* | 19 |
| GA-4688 | GA GOI 687 | *Penicillium oxalicum* | 20 |

The AA used are listed in the following table:

| **Name** | **Abbr.** | **Source organism** | **SEQ ID NO:** |
|---|---|---|---|
| AA-1704 | AcAA | *Asperpillus clavatus* | 21 |
| AA-1708 | AtAA | *Aspergillus terreus* | 22 |
| AA-2115 | AfuAmy1 | *Aspergillus fumigatus Af293* | 23 |
| AA-2205 | NfiAmy1 | *Neosartorya fischeri* | 24 |
| AA-2285 | TemAmy1 | *Talaromyces emersonii* | 25 |
| AA-2301 | PfuAmy1 | *Penecillium funiculosum* | 26 |
| AA-2303 | PfuAmy3 | *Penecillium funiculosum* | 27 |
| AA-2506 | ApuAmy1 | *Aureobasidium pullulans* | 28 |
| AA-2522 | LstAmy1 | *Lipomyces starkeyi* | 29 |
| AA-2676 | OsaAmy2 | *Oryza sativa Japonica Group* | 30 |
| AA-2940 | AacAmy2 | *Asperpillus aculeatus* | 31 |
| AA-3238 | TleAmy1 | *Talaromyces leycettanus* | 32 |
| AA-3239 | TauAmy1 | *Thermoascus_aurantiacus* | 33 |
| AA-3937 | BhaAmv3 | *Brevibacterium halotolerans strain XFB-BI* | 34 |

For the analyses, a slurry of 29.4% dry solids (wt/wt) was made by adding 50%/50% tap water/demineralized water to corn flour substrate (Azure farm Corn Flour organic (FL131) - Azure standard, Dufur Oregon, USA). The pH was adjusted as specified with H₂SO₄ and afterwards urea was added to a final concentration of 500 ppm. Finally, 0.001% w/w PERMGEN 2.5x^{™} protease (DuPont) and 0.1% w/w active dry yeast (Fermentis, France - Ethanol Red) were added. The substrate including the protease and the yeast was divided into the SSF vessels and the selected GA/AA enzyme blend was added (0.107 mg/g ds of GA and 0.016 mg/g ds of AA) to each vessel as well. The vessels were incubated at 32°C and samples were collected at three different time points (*i.e.,* 24 h, 48 h, and 96 h) to analyze sugar, glycerol, and ethanol content using HPLC.

For Examples 2 and 3, the substrate used in the model system screening was 1% (w/w) corn starch (Sigma, Cat. No. S4126) in 50 mM sodium acetate buffer. α-amylase and glucoamylase were combined at the same protein ratio to that of STARGEN^{™} 002 (*i.e.,* AkAA:TrGA=1:6.6). For α-amylase (AA) screening, *Trichoderma reesei* glucoamylase (TrGA; SEQ ID NO: 1) was used as the glucoamylase component and *Aspergillus kawachii* α-amylase (AkAA) (SEQ ID NO: 2) was the benchmark AA. For glucoamylase screening, AkAA was used as the AA component and TrGA was the benchmark GA. The reaction was initiated by adding 10 µL of glucoamylase and 10 µL of α-amylase to 150 µL of the substrate, with final dosages at 10 ppm and 1.5 ppm for GA and AA, respectively. The incubations were done in iEMS (32°C; 900 rpm) for 6, 20 and 28 h, respectively. To quench the reaction, 50 µL of 0.5 M NaOH was added and mixed vigorously. The plate was then sealed with a BioRad seal and centrifuged at 2500 rpm for 3 min. For HPLC analysis, the supernatant was diluted by a factor of 10 using 5 mM H₂SO₄. The diluted supernatant was filtered and 20 µL of the solution was injected into an Agilent 1200 series HPLC equipped with a refractive index detector. The separation column used was a Phenomenex Rezex-RFQ Fast Fruit column (cat# 00D-0223-K0) with a Phenomenex Rezex ROA Organic Acid guard column (cat# 03B-0138-K0). The mobile phase was 5 mM H₂SO₄, and the flow rate was 1.0 mL/min at 85 °C. The amount of glucose released was used to calculate a Performance Index (PI) ration against benchmark AkAA/TrGA combinations.

For Example 4, HPLC (Agilent Technologies 1200 series) run conditions were as follows. A PHENOMENEX REZEX^{™} RFQ-Fast Acid H+ (8%) column was held at 80oC. The solvent was 0.01 N H2SO4 at an isocratic flow of 1.0 ml/min. Injection volumes were 10 µl. Runtimes were 5.3 min. Refractive index detection was used to detect DP4+, DP3, DP2, DP1, glycerol, and ethanol. Appropriate calibration standards were used for quantification of the components present.

In all cases, performance indices (PI) relative to a reference blend were calculated with respect to glucose release and/or ethanol production. Performance equal to the reference blend was assigned a PI of 1.0. Blends with a PI greater than 1.0 at any analysis time point or pH are listed in the Tables in the following Examples and represent improvements over current combinations and methods. Unless otherwise specified, all measurement used, herein, are weight/weight (wt/wt; w/w).

### Example 2

### Results obtained using different GA

A number of different GA were individually tested in *Aspergillus kawachii* α-amylase (AkAA; SEQ ID NO: 2) blends as described in Example 1. The amount of glucose release following 6, 20, and 28 h of incubation at pH 3.5 and 4.5 was measured and divided by the concentration of glucose released by the reference combination of *Trichoderma reesei* glucoamylase (TrGA; SEQ ID NO: 1) and AkAA. The results for the GA with a PI value greater than 1.0 are shown in the Table, below. 18 GA demonstrated superior performance to TrGA when combined with AkAA, remarkably, in some cases, by two-fold.

| **AA** | **GA** | **pH 3.5** | | | **pH 4.5** | | |
|---|---|---|---|---|---|---|---|
| | | **PI (AkAA+TrGA=1.0)** | | | **PI (AkAA+TrGA=1.0)** | | |
| | | **6 h** | **20 h** | **28 h** | **6 h** | **20 h** | **28 h** |
| AkAA | GA-1805 | 1.47 | 1.36 | 1.18 | 1.55 | 1.42 | 1.37 |
| | GA-2040 | 1.67 | 1.4 | 1.23 | 1.64 | 1.43 | 1.41 |
| | GA-2331 | 1.49 | 1.33 | 1.21 | 1.54 | 1.41 | 1.4 |
| | GA-2437 | 1.83 | 1.45 | 1.27 | 1.7 | 1.46 | 1.46 |
| | GA-2439 | 1.94 | 1.49 | 1.28 | 1.97 | 1.56 | 1.52 |
| | GA-2441 | 1.76 | 1.45 | 1.28 | 1.78 | 1.5 | 1.46 |
| | GA-2442 | 1.24 | 1.16 | 1.09 | 1.63 | 1.44 | 1.4 |
| | GA-2578 | 1.84 | 1.47 | 1.28 | 1.58 | 1.46 | 1.37 |
| | GA-2722 | 1.62 | 1.37 | 1.16 | 1.5 | 1.42 | 1.31 |
| | GA-3275 | 1.85 | 1.39 | 1.19 | 1.9 | 1.57 | 1.44 |
| | GA-3280 | 1.86 | 1.42 | 1.21 | 1.66 | 1.48 | 1.4 |
| | GA-3283 | 1.82 | 1.43 | 1.19 | 1.71 | 1.51 | 1.44 |
| | GA-3294 | 1.6 | 1.36 | 1.19 | 1.54 | 1.42 | 1.36 |
| | GA-3298 | 1.86 | 1.46 | 1.23 | 1.65 | 1.47 | 1.39 |
| | GA-3301 | 1.78 | 1.44 | 1.22 | 1.78 | 1.53 | 1.41 |
| | GA-3317 | 2.03 | 1.49 | 1.22 | 1.55 | 1.49 | 1.36 |
| | GA-4686 | 1.6 | 1.36 | 1.19 | 1.67 | 1.45 | 1.36 |
| | GA-4688 | 1.72 | 1.41 | 1.22 | 1.8 | 1.51 | 1.41 |

### Example 3

### Results obtained using different AA

A number of different AA were individually tested in TrGA blends as described in Example 1. The amount of glucose released following 6, 20, and 28 h of incubation at pH 3.5 and 4.5 was measured and divided by the glucose released by the reference combination of TrGA and AkAA. The results for the AA with a PI value greater than 1.0 are shown in the Table, below. Nineteen demonstrated superior performance to AkAA when combined with TrGA, although the improvement was less pronounced than in the case of using different GA in Example 2.

| **GA** | **AA** | **pH 3.5** | | | **pH 4.5** | | |
|---|---|---|---|---|---|---|---|
| | | **PI (AkAA+TrGA=1.0)** | | | **PI (AkAA+TrGA=1.0)** | | |
| | | **6 h** | **20 h** | **28 h** | **6 h** | **20 h** | **28 h** |
| TrGA | AA-1708 | 1.44 | 1.26 | 1.1 | 1.46 | 1.39 | 1.36 |
| | AA-2115 | 0.91 | 0.58 | 0.52 | 1.44 | 1.37 | 1.33 |
| | AA-2205 | 0.61 | 0.38 | 0.34 | 1.41 | 1.35 | 1.33 |
| | AA-2285 | 1.23 | 1.19 | 1.04 | 1.14 | 1.18 | 1.16 |
| | AA-2301 | 0.9 | 0.72 | 0.67 | 1.3 | 1.26 | 1.17 |
| | AA-2303 | 1.15 | 1.12 | 1.01 | 1.14 | 1.19 | 1.13 |
| | AA-2506 | 1.3 | 1.05 | 0.93 | 1.53 | 1.4 | 1.29 |
| | AA-2522 | 1.14 | 1.06 | 0.97 | 1.08 | 1.08 | 1.05 |
| | AA-2676 | 0.53 | 0.3 | 0.26 | 1.35 | 1.26 | 1.16 |
| | AA-2940 | 1.22 | 1.15 | 0.99 | 1.26 | 1.31 | 1.2 |
| | AA-3238 | 1.27 | 1.22 | 1.13 | 1.31 | 1.35 | 1.26 |
| | AA-3239 | 1.13 | 1 | 0.96 | 1.18 | 1.16 | 1.13 |
| | AA-3937 | 0.79 | 0.41 | 0.36 | 1.47 | 1.42 | 1.34 |
| | AA-1704 | 1.24 | 1.1 | 1.09 | 1.35 | 1.3 | 1.25 |
| | AkAA | 1 | 1 | 1 | 1 | 1 | 1 |

### Example 4

### Identification of high performing GA/AA blends

A number of different AA/GA blends were tested as described in Example 1. The concentration of ethanol following 24, 48, and 96 h of incubation at pH 3.5 was measured, averaged, and divided by the concentration of ethanol produced by the reference combination of TrGA and AkAA.

Blends with a PI greater than 1.0 are listed in the following Table.

| **AA** | **GA** | **PI** |
|---|---|---|
| AA-1708 | GA-3317 | 1.32 |
| | GA-3298 | 1.3 |
| | GA-2040 | 1.22 |
| | GA-3280 | 1.21 |
| | GA-2441 | 1.2 |
| | GA-1805 | 1.2 |
| | GA-2439 | 1.18 |
| | GA-4686 | 1.15 |
| | GA-3301 | 1.15 |
| | GA-2331 | 1.14 |
| | GA-3275 | 1.11 |
| AA-3238 | GA-3317 | 1.19 |
| | GA-3280 | 1.17 |
| | GA-3298 | 1.16 |
| | GA-4688 | 1.16 |
| | GA-2441 | 1.15 |
| | GA-4686 | 1.11 |
| | GA-2040 | 1.10 |
| AA-2285 | GA-3317 | 1.14 |
| | GA-2441 | 1.13 |
| | GA-3298 | 1.12 |
| | GA-3280 | 1.10 |
| AA-2522 | GA-3317 | 1.11 |
| | GA-3298 | 1.10 |
| | GA-2439 | 1.10 |
| AA-3239 | GA-3298 | 1.11 |
| | GA-1805 | 1.10 |
| | GA-3317 | 1.10 |
| | GA-2439 | 1.08 |
| AA-2303 | GA-3298 | 1.15 |
| | GA-3317 | 1.14 |
| | GA-2439 | 1.13 |
| | GA-3301 | 1.12 |
| | GA-2441 | 1.10 |
| | GA-3280 | 1.08 |
| AA-2940 | GA-3317 | 1.12 |
| AA-1704 | GA-3298 | 1.08 |

Insofar as the product referred to by a trademark name varies with time, the product having the characteristics described in the relevant product literature, including websites, from the manufacturer as of the effective filing date of the application is intended. The headings provided herein are not limitations of the various aspects or embodiments of the invention, which can be had by reference to the specification as a whole. Although preferred methods and materials have been described, any methods and materials similar or equivalent to those described herein can be used in the practice or testing of the present invention. Unless otherwise apparent from the context, any embodiment, aspect, step, feature, element or limitation can be used in combination with any other.

## Claims

1. A method for processing granular starch comprising:
contacting a slurry comprising granular starch with a glucoamylase and a granular starch-converting α-amylase, at a temperature at or below the gelatinization temperature of the granular starch, to produce saccharides fermentable by a fermenting organism; wherein:
(i) the granular starch-converting α-amylase comprises an amino acid sequence having at least 95% amino acid sequence identity to SEQ ID NO: 22, and the glucoamylase has at least 95% amino acid sequence identity to any one of SEQ ID NOs: 18, 16, 4, 13, 8, 3, 7, 19, 17, 5, and 12;
(ii) the granular starch-converting α-amylase comprises an amino acid sequence having at least 95% amino acid sequence identity to SEQ ID NO: 27, and the glucoamylase has at least 95% amino acid sequence identity to any one of SEQ ID NOs: 16, 18, 7, 17, 8, and 13; or
(iii) the granular starch-converting α-amylase comprises an amino acid sequence having at least 95% amino acid sequence identity to SEQ ID NO: 32, and the glucoamylase has at least 95% amino acid sequence identity to any one of SEQ ID NOs: 18, 13, 16, 20, 8, 19, and 4;
wherein contacting the slurry with the glucoamylase and the granular starch-converting α-amylase results in increased starch conversion, increased glucose release or in increased total glucose equivalents compared to contacting the same slurry with the same glucoamylase and α-amylase from *Aspergillus kawachii* (AkAA) having the amino acid sequence of SEQ ID NO: 2.

2. The method of claim 1, wherein the increased total glucose equivalents is at least 5% higher, and preferably at least 10% higher, compared to the amount produced by contacting the same slurry with the glucoamylase and α-amylase from *Aspergillus kawachii* (AkAA) having the amino acid sequence of SEQ ID NO: 2.

3. The method of any of the preceding claims, wherein the method results in the production of glucose, maltose, oligosaccharides, or a mixture thereof, optionally in the form of a syrup.

4. The method of any of the preceding claims, further comprising contacting the saccharides with a fermenting organism to produce an end of fermentation product; wherein the contacting results in increased production of an end of fermentation product compared to contacting the same slurry with the glucoamylase and α-amylase from *Aspergillus kawachii* (AkAA) having the amino acid sequence of SEQ ID NO: 2.

5. The method of claim 4, wherein the end of fermentation product is ethanol or a non-ethanol biochemical.

6. The method of any of claims 1-5, wherein the glucoamylase and the granular starch-converting α-amylase are added simultaneously.

7. The method of any of claims 4 or 5, wherein the glucoamylase and/or the granular starch-converting α-amylase and the fermenting organism are added simultaneously.

8. The method of any of claims 1-7, wherein the glucoamylase and/or the granular starch-converting α-amylase are produced by a fermenting organism.

9. The method of any of the preceding claims, further comprising the addition of an additional enzyme to the slurry.

10. A composition comprising a granular starch-converting α-amylase in combination with a glucoamylase, wherein:
(i) the granular starch-converting α-amylase comprises an amino acid sequence having at least 95% amino acid sequence identity to SEQ ID NO: 22, and the glucoamylase has at least 95% amino acid sequence identity to any one of SEQ ID NOs: 18, 16, 4, 13, 8, 3, 7, 19, 17, 5, and 12;
(ii) the granular starch-converting α-amylase comprises an amino acid sequence having at least 95% amino acid sequence identity to SEQ ID NO: 27, and the glucoamylase has at least 95% amino acid sequence identity to any one of SEQ ID NOs: 16, 18, 7, 17, 8, and 13; or
(iii) the granular starch-converting α-amylase comprises an amino acid sequence having at least 95% amino acid sequence identity to SEQ ID NO: 32, and the glucoamylase has at least 95% amino acid sequence identity to any one of SEQ ID NOs: 18, 13, 16, 20, 8, 19, and 4.

11. A fermenting organism capable of producing a combination of a granular starch-converting α-amylase and a glucoamylase, wherein:
(i) the granular starch-converting α-amylase comprises an amino acid sequence having at least 95% amino acid sequence identity to SEQ ID NO: 22, and the glucoamylase has at least 95% amino acid sequence identity to any one of SEQ ID NOs: 18, 16, 4, 13, 8, 3, 7, 19, 17, 5, and 12;
(ii) the granular starch-converting α-amylase comprises an amino acid sequence having at least 95% amino acid sequence identity to SEQ ID NO: 27, and the glucoamylase has at least 95% amino acid sequence identity to any one of SEQ ID NOs: 16, 18, 7, 17, 8, and 13; or
(iii) the granular starch-converting α-amylase comprises an amino acid sequence having at least 95% amino acid sequence identity to SEQ ID NO: 32, and the glucoamylase has at least 95% amino acid sequence identity to any one of SEQ ID NOs: 18, 13, 16, 20, 8, 19, and 4.

## Patentansprüche

1. Verfahren zur Verarbeitung von körniger Stärke, umfassend:
In-Kontakt-Bringen einer körnige Stärke umfassenden Aufschlämmung mit einer Glucoamylase und einer körnige Stärke umwandelnden α-Amylase bei einer Temperatur bei oder unterhalb der Verkleisterungstemperatur der körnigen Stärke zur Erzeugung von Sacchariden, die durch einen fermentierenden Organismus fermentierbar sind; wobei:
(i) die körnige Stärke umwandelnde α-Amylase eine Aminosäuresequenz mit einer Aminosäuresequenzidentität von mindestens 95 % mit SEQ ID NO: 22 umfasst und die Glucoamylase eine Aminosäuresequenzidentität von mindestens 95 % mit einer von SEQ ID NO: 18, 16, 4, 13, 8, 3, 7, 19, 17, 5 und 12 aufweist;
(ii) die körnige Stärke umwandelnde α-Amylase eine Aminosäuresequenz mit einer Aminosäuresequenzidentität von mindestens 95 % mit SEQ ID NO: 27 umfasst und die Glucoamylase eine Aminosäuresequenzidentität von mindestens 95 % mit einer von SEQ ID NO: 16, 18, 7, 17, 8 und 13 aufweist; oder
(iii) die körnige Stärke umwandelnde α-Amylase eine Aminosäuresequenz mit einer Aminosäuresequenzidentität von mindestens 95 % mit SEQ ID NO: 32 umfasst und die Glucoamylase eine Aminosäuresequenzidentität von mindestens 95 % mit einer von SEQ ID NO: 18, 13, 16, 20, 8, 19 und 4 aufweist;
wobei das In-Kontakt-Bringen der Aufschlämmung mit der Glucoamylase und der körnige Stärke umwandelnden α-Amylase zu erhöhter Stärkeumwandlung, erhöhter Glucosefreisetzung oder einer erhöhten Gesamtzahl von Glucose-Äquivalenten im Vergleich zum In-Kontakt-Bringen der gleichen Aufschlämmung mit der gleichen Glucoamylase und α-Amylase aus Aspergillus kawachii (AkAA) mit der Aminosäuresequenz unter SEQ ID NO: 2 führt.

2. Verfahren nach Anspruch 1, wobei die erhöhte Gesamtzahl von Glucose-Äquivalenten mindestens 5 % höher und bevorzugt mindestens 10 % höher ist im Vergleich zur Menge, die durch In-Kontakt-Bringen der gleichen Aufschlämmung mit der Glucoamylase und α-Amylase aus Aspergillus kawachii (AkAA) mit der Aminosäuresequenz unter SEQ ID NO: 2 erzeugt wird.

3. Verfahren nach einem der vorhergehenden Ansprüche, wobei das Verfahren zur Erzeugung von Glucose, Maltose, Oligosacchariden oder einer Mischung davon, gegebenenfalls in Form eines Sirups, führt.

4. Verfahren nach einem der vorhergehenden Ansprüche, ferner umfassend In-Kontakt-Bringen der Saccharide mit einem fermentierenden Organismus zur Erzeugung eines Fermentationsendprodukts; wobei das In-Kontakt-Bringen zu einer erhöhten Erzeugung eines Fermentationsendprodukts im Vergleich zum In-Kontakt-Bringen der gleichen Aufschlämmung mit der Glucoamylase und α-Amylase aus Aspergillus kawachii (AkAA) mit der Aminosäuresequenz unter SEQ ID NO: 2 führt.

5. Verfahren nach Anspruch 4, wobei es sich bei dem Fermentationsendprodukt um Ethanol oder eine Nicht-Ethanol-Biochemikalie handelt.

6. Verfahren nach einem der Ansprüche 1-5, wobei die Glucoamylase und die körnige Stärke umwandelnde α-Amylase gleichzeitig zugegeben werden.

7. Verfahren nach Anspruch 4 oder 5, wobei die Glucoamylase und/oder die körnige Stärke umwandelnde α-Amylase und der fermentierende Organismus gleichzeitig zugegeben werden.

8. Verfahren nach einem der Ansprüche 1-7, wobei die Glucoamylase und/oder die körnige Stärke umwandelnde α-Amylase durch einen fermentierenden Organismus erzeugt werden.

9. Verfahren nach einem der vorhergehenden Ansprüche, ferner umfassend die Zugabe eines zusätzlichen Enzyms zur Aufschlämmung.

10. Zusammensetzung, umfassend eine körnige Stärke umwandelnde α-Amylase in Kombination mit einer Glucoamylase, wobei:
(i) die körnige Stärke umwandelnde α-Amylase eine Aminosäuresequenz mit einer Aminosäuresequenzidentität von mindestens 95 % mit SEQ ID NO: 22 umfasst und die Glucoamylase eine Aminosäuresequenzidentität von mindestens 95 % mit einer von SEQ ID NO: 18, 16, 4, 13, 8, 3, 7, 19, 17, 5 und 12 aufweist;
(ii) die körnige Stärke umwandelnde α-Amylase eine Aminosäuresequenz mit einer Aminosäuresequenzidentität von mindestens 95 % mit SEQ ID NO: 27 umfasst und die Glucoamylase eine Aminosäuresequenzidentität von mindestens 95 % mit einer von SEQ ID NO: 16, 18, 7, 17, 8 und 13 aufweist; oder
(iii) die körnige Stärke umwandelnde α-Amylase eine Aminosäuresequenz mit einer Aminosäuresequenzidentität von mindestens 95 % mit SEQ ID NO: 32 umfasst und die Glucoamylase eine Aminosäuresequenzidentität von mindestens 95 % mit einer von SEQ ID NO: 18, 13, 16, 20, 8, 19 und 4 aufweist.

11. Fermentierender Organismus, fähig zur Erzeugung einer Kombination aus einer körnige Stärke umwandelnden α-Amylase und einer Glucoamylase, wobei:
(i) die körnige Stärke umwandelnde α-Amylase eine Aminosäuresequenz mit einer Aminosäuresequenzidentität von mindestens 95 % mit SEQ ID NO: 22 umfasst und die Glucoamylase eine Aminosäuresequenzidentität von mindestens 95 % mit einer von SEQ ID NO: 18, 16, 4, 13, 8, 3, 7, 19, 17, 5 und 12 aufweist;
(ii) die körnige Stärke umwandelnde α-Amylase eine Aminosäuresequenz mit einer Aminosäuresequenzidentität von mindestens 95 % mit SEQ ID NO: 27 umfasst und die Glucoamylase eine Aminosäuresequenzidentität von mindestens 95 % mit einer von SEQ ID NO: 16, 18, 7, 17, 8 und 13 aufweist; oder
(iii) die körnige Stärke umwandelnde α-Amylase eine Aminosäuresequenz mit einer Aminosäuresequenzidentität von mindestens 95 % mit SEQ ID NO: 32 umfasst und die Glucoamylase eine Aminosäuresequenzidentität von mindestens 95 % mit einer von SEQ ID NO: 18, 13, 16, 20, 8, 19 und 4 aufweist.

## Revendications

1. Procédé de traitement d'amidon granulaire comprenant :
la mise en contact d'une suspension comprenant de l'amidon granulaire avec une glucoamylase et une α-amylase convertissant l'amidon granulaire, à une température à ou en dessous de la température de gélatinisation de l'amidon granulaire, pour produire des saccharides fermentescibles par un organisme de fermentation ; dans lequel :
(i) la α-amylase convertissant l'amidon granulaire comprend une séquence d'acides aminés possédant au moins 95 % d'identité de séquence d'acides aminés avec SEQ ID NO: 22, et la glucoamylase possède au moins 95% d'identité de séquence en acides aminés avec l'une quelconque des SEQ ID NO: 18, 16, 4, 13, 8, 3, 7, 19, 17, 5, et 12 ;
(ii) la α-amylase convertissant l'amidon granulaire comprend une séquence d'acides aminés possédant au moins 95 % d'identité de séquence d'acides aminés avec SEQ ID NO: 27, et la glucoamylase possède au moins 95% d'identité de séquence en acides aminés avec l'une quelconque des SEQ ID NO: 16, 18, 7, 17, 8, et 13 ; ou
(iii) la α-amylase convertissant l'amidon granulaire comprend une séquence d'acides aminés possédant au moins 95 % d'identité de séquence d'acides aminés avec SEQ ID NO: 32, et la glucoamylase possède au moins 95% d'identité de séquence en acides aminés avec l'une quelconque des SEQ ID NO: 18, 13, 16, 20, 8, 19 et 4 ;
dans lequel la mise en contact de la suspension avec la glucoamylase et la α-amylase convertissant l'amidon granulaire a pour effet une conversion accrue de l'amidon, une augmentation de la libération de glucose ou augmentation des équivalents totaux de glucose par rapport à la mise en contact de la même suspension avec la même glucoamylase et α-amylase d'Aspergillus kawachii (AkAA) ayant la séquence d'acides aminés de SEQ ID NO: 2.

2. Procédé selon la revendication 1, dans lequel les équivalents totaux de glucose augmentés sont supérieurs d'au moins 5 %, et de préférence d'au moins 10 %, par rapport à la quantité produite par mise en contact de la même suspension avec la glucoamylase et la α-amylase d'Aspergillus kawachii (AkAA) ayant la séquence d'acides aminés de SEQ ID NO: 2.

3. Procédé selon l'une quelconque des revendications précédentes, dans lequel le procédé entraîne la production de glucose, de maltose, d'oligosaccharides ou d'un mélange de ceux-ci, éventuellement sous la forme d'un sirop.

4. Procédé selon l'une quelconque des revendications précédentes, comprenant en outre la mise en contact des saccharides avec un organisme de fermentation pour produire un produit de fin de fermentation ; dans lequel la mise en contact a pour résultat une production accrue d'un produit de fin de fermentation par rapport à la mise en contact de la même suspension avec la glucoamylase et la α-amylase d'Aspergillus kawachii (AkAA) ayant la séquence d'acides aminés de SEQ ID NO: 2.

5. Procédé selon la revendication 4, dans lequel le produit de fin de fermentation est l'éthanol ou un produit biochimique non-éthanol.

6. Procédé selon l'une quelconque des revendications 1 à 5, dans lequel la glucoamylase et l'α-amylase convertissant l'amidon granulaire sont ajoutées simultanément.

7. Procédé selon l'une quelconque des revendications 4 ou 5, dans lequel la glucoamylase et/ou l'α-amylase convertissant l'amidon granulaire et l'organisme de fermentation sont ajoutés simultanément.

8. Procédé selon l'une quelconque des revendications 1 à 7, dans lequel la glucoamylase et/ou l'α-amylase convertissant l'amidon granulaire sont produites par un organisme de fermentation.

9. Procédé selon l'une quelconque des revendications précédentes, comprenant en outre l'ajout d'une enzyme supplémentaire à la suspension.

10. Composition comprenant une α-amylase convertissant l'amidon granulaire en combinaison avec une glucoamylase, dans laquelle :
(i) la α-amylase convertissant l'amidon granulaire comprend une séquence d'acides aminés possédant au moins 95 % d'identité de séquence d'acides aminés avec SEQ ID NO: 22, et la glucoamylase possède au moins 95% d'identité de séquence en acides aminés avec l'une quelconque des SEQ ID NO: 18, 16, 4, 13, 8, 3, 7, 19, 17, 5, et 12 ;
(ii) la α-amylase convertissant l'amidon granulaire comprend une séquence d'acides aminés possédant au moins 95 % d'identité de séquence d'acides aminés avec SEQ ID NO: 27, et la glucoamylase possède au moins 95% d'identité de séquence en acides aminés avec l'une quelconque des SEQ ID NO: 16, 18, 7, 17, 8, et 13 ; ou
(iii) la α-amylase convertissant l'amidon granulaire comprend une séquence d'acides aminés possédant au moins 95 % d'identité de séquence d'acides aminés avec SEQ ID NO: 32, et la glucoamylase possède au moins 95% d'identité de séquence en acides aminés avec l'une quelconque des SEQ ID NO: 18, 13, 16, 20, 8, 19 et 4.

11. Organisme de fermentation capable de produire une combinaison d'une α-amylase convertissant l'amidon granulaire et d'une glucoamylase, dans lequel :
(i) la α-amylase convertissant l'amidon granulaire comprend une séquence d'acides aminés possédant au moins 95 % d'identité de séquence d'acides aminés avec SEQ ID NO: 22, et la glucoamylase possède au moins 95% d'identité de séquence en acides aminés avec l'une quelconque des SEQ ID NO: 18, 16, 4, 13, 8, 3, 7, 19, 17, 5, et 12 ;
(ii) la α-amylase convertissant l'amidon granulaire comprend une séquence d'acides aminés possédant au moins 95 % d'identité de séquence d'acides aminés avec SEQ ID NO: 27, et la glucoamylase possède au moins 95% d'identité de séquence en acides aminés avec l'une quelconque des SEQ ID NO: 16, 18, 7, 17, 8, et 13 ; ou
(iii) la α-amylase convertissant l'amidon granulaire comprend une séquence d'acides aminés possédant au moins 95 % d'identité de séquence d'acides aminés avec SEQ ID NO: 32, et la glucoamylase possède au moins 95% d'identité de séquence en acides aminés avec l'une quelconque des SEQ ID NO: 18, 13, 16, 20, 8, 19 et 4.
